# EUROPÄISCHE PATENTSCHRIFT

(11) **EP 3 937 854 B1**
(45) Veröffentlichungstag und Bekanntmachung des Hinweises auf die Patenterteilung: **22.10.2025**
(21) Anmeldenummer: 20712270.6
(22) Anmeldetag: 13.03.2020
(51) Int. Cl.: A61F 2/38

(54) **KNIEGELENKENDOPROTHESENVORRICHTUNG UND KNIEGELENKENDOPROTHESE**
KNEE-JOINT ENDOPROSTHESIS DEVICE AND KNEE-JOINT ENDOPROSTHESIS
DISPOSITIF D'ENDOPROTHÈSE DE L'ARTICULATION DU GENOU ET ENDOPROTHÈSE DE L'ARTICULATION DU GENOU

(30) Priorität: 15.03.2019 DE 102019106599
(43) Veröffentlichungstag der Anmeldung: 19.01.2022
(73) Patentinhaber: Aesculap AG, 78532 Tuttlingen (DE)
(72) Erfinder: BOLLINGER, Arthur, 78234 Engen (DE)
(74) Vertreter: Hoeger, Stellrecht & Partner Patentanwälte mbB
(86) Internationale Anmeldenummer: PCT/EP2020/056768
(87) Internationale Veröffentlichungsnummer: WO 2020/187722

(56) Entgegenhaltungen:
- EP-A2- 1 374 805
- EP-A2- 1 674 052
- US-A1- 2008 306 603

## Beschreibung

Die vorliegende Erfindung betrifft eine Kniegelenkendoprothesenvorrichtung umfassend eine Tibiakomponente zum Verankern an einer Tibia und eine Meniskuskomponente, wobei die Tibiakomponente und die Meniskuskomponente in einer Kopplungsstellung miteinander gekoppelt und in einer Trennstellung vollständig voneinander getrennt sind, welche Tibiakomponente eine Anlageebene definierende Oberseite aufweist, an welcher eine Unterseite der Meniskuskomponente in der Kopplungsstellung anliegt, wobei die Kniegelenkendoprothesenvorrichtung von der Trennstellung durch eine Relativbewegung der Tibiakomponente und der Meniskuskomponente in die Kopplungsstellung überführbar ist.

Ferner betrifft die vorliegende Erfindung eine Kniegelenkendoprothese umfassend eine Kniegelenkendoprothesenvorrichtung und eine mit dieser zusammenwirkende Femurkomponente.

Kniegelenkendoprothesen der eingangs beschriebenen Art sind in verschiedenen Varianten bekannt. In der Regel werden zunächst die Femurkomponente und die Tibiakomponente der Kniegelenkendoprothese an der Tibia beziehungsweise dem Femur eines Patienten verankert und dann die Meniskuskomponente zwischen diese eingebracht. Insbesondere dann, wenn die Implantation der Kniegelenkendoprothese unter Erhaltung von das Kniegelenk stabilisierenden Seidenbändern erfolgt, ist bei bekannten Lösungen stets eine Überdehnung dieser Bänder Voraussetzung, um die Meniskuskomponente zwischen die Tibiakomponente und die Femurkomponente einzubringen. Dies ist insbesondere unabhängig davon, ob die Meniskuskomponente unbeweglich an der Tibia befestigt wird oder an dieser rotierbar gelagert werden soll. Die Überdehnung der Bänder ist nach Möglichkeit jedoch zu vermeiden, um eine Stabilität des Knies des Patienten nach der Implantation der Kniegelenkendoprothese nicht zu beeinträchtigen.

Aus der US 2008/0306603 A1 sind ein modularer Implantatteil und eine Kniegelenkprothese bekannt. Eine modulare Kniegelenkprothese ist in der EP 1 374 805 A2 beschrieben. Die EP 1 674 052 A2 befasst sich mit Knieprothesen.

Es ist daher eine Aufgabe der vorliegenden Erfindung, eine Kniegelenkendoprothesenvorrichtung sowie eine Kniegelenkendoprothese der eingangs beschriebenen Art so zu verbessern, dass die Meniskuskomponente und die Tibiakomponente bandschonend miteinander gekoppelt werden können.

Diese Aufgabe wird bei einer Kniegelenkendoprothesenvorrichtung der eingangs beschriebenen Art erfindungsgemäß dadurch gelöst, dass die Kniegelenkendoprothesenvorrichtung eine Kopplungseinrichtung umfasst zum Koppeln der Tibiakomponente und der Meniskuskomponente in der Kopplungsstellung, dass die Kopplungseinrichtung derart ausgebildet ist, dass die Tibiakomponente und die Meniskuskomponente beim Überführen der Kniegelenkendoprothesenvorrichtung von der Trennstellung in die Kopplungsstellung in einer Richtung parallel zur Anlageebene relativ zueinander von der Trennstellung in eine Zwischenstellung und von der Zwischenstellung in einer Richtung quer, insbesondere senkrecht, zur Anlageebene und aufeinander zu in die Kopplungsstellung verschiebbar sind.

Die erfindungsgemäß vorgeschlagene Weiterbildung bekannter Kniegelenkendoprothesenvorrichtungen ermöglicht es insbesondere, die Meniskuskomponente nach Implantation der Femurkomponente und der Tibiakomponente von vorne, also von anterior her kommend, zwischen diese einzuschieben, insbesondere ohne oder nur mit einer sehr geringen Aufdehnung der bei der Implantation erhaltenen Seitenbänder. Durch die Möglichkeit, die Meniskuskomponente insbesondere parallel zur Anlageebene relativ zur Tibiakomponente zu verschieben, kann diese von vorne und damit sowohl die Bänder als auch den Patienten schonend eingesetzt werden. Insbesondere kann diese Ausbildung der Kniegelenkendoprothesenvorrichtung sowohl für Kniegelenkendoprothesen eingesetzt werden, bei denen die Meniskuskomponente in der Kopplungsstellung relativ zur Tibiakomponente unbeweglich gehalten oder relativ zu dieser verdreh- und/oder verschiebbar angeordnet ist. Ferner lässt sich die Meniskuskomponente bei einer wie vorgeschlagen weitergebildeten Kniegelenkendoprothesenvorrichtung bei Bedarf auch wieder auf einfache Weise auswechseln, beispielsweise wenn diese beschädigt ist oder eine vom Operateur gewählte Meniskuskomponente zu hoch oder zu niedrig ist.

Günstig ist es, wenn die die Kopplungseinrichtung mindestens ein erstes Kopplungselement und mindestens ein zweites Kopplungselement umfasst, wenn das mindestens eine erste Kopplungselement an der Tibiakomponente oder an der Meniskuskomponente angeordnet oder ausgebildet ist, wenn das mindestens eine zweite Kopplungselement an der Meniskuskomponente oder an der Tibiakomponente angeordnet oder ausgebildet ist, wenn das mindestens eine erste Kopplungselement und das mindestens eine zweite Kopplungselement in der Trennstellung außer Eingriff stehen und wenn das mindestens eine erste Kopplungselement und das mindestens eine zweite Kopplungselement in der Kopplungsstellung in Eingriff stehen. Eine derartige Kopplungseinrichtung lässt sich auf einfache Weise ausbilden und erleichtert insbesondere das Koppeln der Meniskuskomponente und der Tibiakomponente miteinander.

Vorteilhaft ist es, wenn das mindestens eine erste Kopplungselement in Form eines Kopplungsvorsprungs ausgebildet ist, wenn das mindestens eine zweite Kopplungselement in Form einer Kopplungsaufnahme mit einer Kopplungsausnehmung ausgebildet ist, wenn die Kopplungsaufnahme ausgebildet ist zum Aufnehmen des Kopplungsvorsprungs in der Zwischenstellung und wenn die Kopplungsausnehmung ausgebildet ist zum Aufnehmen mindestens eines Teils des Kopplungsvorsprungs in der Kopplungsstellung. Die vorgeschlagene Gestaltung der mindestens einen ersten und zweiten Kopplungselemente ermöglicht auf einfache Weise das in Eingriff Bringen und Bewegen der Meniskuskomponente und der Tibiakomponente relativ zueinander aus der Trennstellung in die Zwischenstellung. Ferner ermöglicht es die Kopplungsausnehmung, einen Teil des Kopplungsvorsprungs in der Kopplungsstellung aufzunehmen. Insbesondere ist dies möglich durch eine Bewegung des mindestens einen Teils des Kopplungsvorsprungs von der Zwischenstellung in die Kopplungsausnehmung hinein, so dass die Kniegelenkendoprothesenvorrichtung die Kopplungsstellung einnimmt. Dies kann insbesondere erreicht werden, indem die Kopplungsausnehmung in Form einer an der Kopplungsaufnahme von der Anlageebene weg weisenden Vertiefung oder Hinterschneidung ausgebildet ist. Zudem können durch die Kopplungsausnehmung die Meniskuskomponente und die Tibiakomponente beim Übergang von der Zwischenstellung in die Kopplungsstellung etwas aufeinander zu bewegt werden.

Auf einfache Weise lässt sich die Meniskuskomponente an der Tibiakomponente in der Kopplungsstellung sichern, wenn die Kopplungsausnehmung in einer Richtung senkrecht zur Anlageebene geöffnet ist. Insbesondere können so die Meniskuskomponente und die Tibiakomponente beim Übergang von der Zwischenstellung in die Kopplungsstellung senkrecht zur Anlageebene aufeinander zu bewegt werden.

Vorzugsweise ist die Kopplungsausnehmung ausschließlich in einer Richtung senkrecht zur Anlageebene geöffnet. So kann insbesondere sichergestellt werden, dass die Bewegungen der Meniskuskomponente und der Tibiakomponente relativ zueinander von der Trennstellung in die Zwischenstellung einerseits und von der Zwischenstellung in die Kopplungsstellung andererseits im mathematischen Sinn linear unabhängig voneinander sind. Dies ermöglicht insbesondere eine Sicherung der Kniegelenkendoprothesenvorrichtung in der Kopplungsstellung derart, dass die Meniskuskomponente und die Tibiakomponente relativ zueinander nicht voneinander lösbar sind nur durch eine Relativbewegung parallel zur Anlageebene. Es ist ferner noch eine Bewegung quer zur Anlageebene erforderlich, um die Kniegelenkendoprothesenvorrichtung zunächst von der Kopplungsstellung in die Zwischenstellung zu überführen.

Vorteilhaft ist es, wenn die Meniskuskomponente die Kopplungsausnehmung umfasst, die in Richtung auf die Tibiakomponente hin weisend geöffnet ist zum Einführen des mindestens einen Teils des Kopplungsvorsprungs infolge einer Relativbewegung von der Zwischenstellung in die Kopplungsstellung in der Richtung quer, insbesondere senkrecht, zur Anlageebene. Die Kopplungsausnehmung an der Meniskuskomponente auszubilden hat insbesondere den Vorteil, dass in dem Fall, in dem die Meniskuskomponente aus einem Kunststoff ausgebildet ist, keine fragilen Vorsprünge an dieser ausgebildet werden müssen, um die Meniskuskomponente mit einer vorzugsweise aus einem metallischen Werkstoff ausgebildeten Tibiakomponente zu koppeln. So lässt sich insbesondere die Stabilität der Kniegelenkendoprothesenvorrichtung insgesamt verbessern.

Eine Kopplung der Meniskuskomponente und der Tibiakomponente lässt sich insbesondere dadurch weiter vereinfachen, dass die Meniskuskomponente eine Einführöffnung aufweist zum Einführen des Kopplungsvorsprungs in die Kopplungsaufnahme der Meniskuskomponente hinein. Insbesondere kann die Einführöffnung an der Meniskuskomponente in einem hinteren oder posterioren Bereich der Meniskuskomponente vorgesehen sein, so dass diese von anterior her kommend, also von vorne, auf die Tibiakomponente aufgeschoben werden kann.

Günstig ist es, wenn der Kopplungsvorsprung eine von der Anlageebene weg weisende Kopplungsvorsprungoberseite aufweist, wenn die Einführöffnung eine obere Begrenzungsfläche und eine untere Begrenzungsfläche definiert, die parallel zur Unterseite der Meniskuskomponente verlaufen, wenn ein Abstand der unteren Begrenzungsfläche von der Unterseite der Meniskuskomponente kleiner ist als ein Abstand der oberen Begrenzungsfläche von der Unterseite der Meniskuskomponente und wenn der Abstand der unteren Begrenzungsfläche von der Unterseite der Meniskuskomponente kleiner ist als ein Abstand der Kopplungsvorsprungoberseite von der Anlageebene. Diese Ausgestaltung ermöglicht es insbesondere, die Meniskuskomponente relativ zur Tibiakomponente parallel zur Anlageebene zu verschieben, um die Kniegelenkendoprothesenvorrichtung von der Trennstellung in die Zwischenstellung zu überführen, wobei dabei die Unterseite der Meniskuskomponente von der Oberseite etwas beabstandet ist. Dieser Abstand zwischen der Unterseite der Meniskuskomponente und der Oberseite der Tibiakomponente, den die Kopplungseinrichtung vorsieht, wenn die Tibiakomponente und die Meniskuskomponente von der Trennstellung in die Zwischenstellung überführt werden, ermöglicht dann insbesondere eine Bewegung von der Zwischenstellung in die Kopplungsstellung um genau diesen Abstand derart, dass der Kopplungsvorsprung in die Kopplungsausnehmung eingeführt werden kann.

Günstig ist es, wenn die Einführöffnung eine Einführbreite definiert, wenn der Kopplungsvorsprung eine Kopplungsvorsprungbreite definiert und wenn die Kopplungsvorsprungbreite mindestens der Einführbreite entspricht. Ist der Kopplungsvorsprung so breit, dass die Kopplungsvorsprungbreite der Einführbreite entspricht, können die Meniskuskomponente und die Tibiakomponente im Wesentlichen ohne einen spürbaren Widerstand von der Trennstellung in die Zwischenstellung überführt werden. Ist die Kopplungsvorsprungbreite jedoch größer als die Einführbreite, muss entweder die Einführöffnung etwas aufgeweitet oder der Kopplungsvorsprung etwas in seiner Breite verringert werden beim Überführen der Kniegelenkendoprothesenvorrichtung von der Trennstellung in die Zwischenstellung und umgekehrt. Insbesondere lässt sich auf diese Weise die Meniskuskomponente bereits an der Tibiakomponente gegen ein unbeabsichtigtes Lösen sichern, da dann ein definierter Einführwiderstand überwunden werden muss, um die Meniskuskomponente und die Tibiakomponente wieder voneinander zu trennen. Zudem kann beim Koppeln der Meniskuskomponente und der Tibiakomponente ein Operateur in diesem Fall eine haptische Rückmeldung erhalten, wenn die Kniegelenkendoprothesenvorrichtung die Zwischenstellung einnimmt, also diejenige Stellung, aus der heraus die Kniegelenkendoprothesenvorrichtung durch Relativbewegung der Meniskuskomponente und der Tibiakomponente in einer Richtung quer zur Anlageebene die Kniegelenkendoprothesenvorrichtung in die Kopplungsstellung überführbar ist.

Vorzugsweise ist die Kopplungsvorsprungbreite größer als die Einführbreite. In diesem Fall kann wie bereits beschrieben insbesondere eine Sicherung der Meniskuskomponente und der Tibiakomponente erreicht werden, wenn die Kniegelenkendoprothesenvorrichtung die Zwischenstellung einnimmt und lediglich durch eine Bewegung, also eine Verschiebung, parallel zur Anlageebene von der Zwischenstellung in die Trennstellung überführt werden könnte.

Gemäß einer weiteren bevorzugten Ausführungsform der Kniegelenkendoprothesenvorrichtung kann vorgesehen sein, dass am Kopplungsvorsprung und/oder an der Einführöffnung mindestens erste und/oder zweite bewegliche Kupplungselemente angeordnet oder ausgebildet sind und dass die Kupplungselemente beim Übergang von der Trennstellung in die Zwischenstellung bewegbar sind zum temporären Verkleinern der Kopplungsvorsprungbreite und/oder zum Vergrößern der Einführbreite. Insbesondere kann durch diese besondere Ausgestaltung des Kopplungsvorsprungs und/oder der Einführöffnung eine Rast- oder Schnappverbindung ausgebildet werden, die ein Einschnappen oder Verrasten der Tibiakomponente und der Meniskuskomponente relativ zueinander beim Erreichen der Zwischenstellung ausgehend von der Trennstellung ermöglichen.

Auf einfache und kompakte Weise lässt sich die Kniegelenkendoprothesenvorrichtung ausbilden, wenn die mindestens einen ersten und/oder zweiten Kupplungselemente parallel zur Anlageebene bewegbar angeordnet oder ausgebildet sind. Insbesondere können sie dadurch lediglich eine Bewegung der Meniskuskomponente und der Tibiakomponente relativ zueinander parallel zur Anlageebene beeinflussen, nicht jedoch quer, insbesondere senkrecht, zur Anlageebene.

Günstig ist es, wenn die mindestens einen ersten und/oder zweiten Kupplungselemente entgegen der Wirkung einer Rückstelleinrichtung aus einer Kupplungselementgrundstellung, in welcher die Rückstelleinrichtung keine Rückstellkraft auf die mindestens einen ersten und/oder zweiten Kupplungselemente ausübt, in eine Kupplungselementauslenkstellung auslenkbar sind. Auf diese Weise kann eine Schnapp- und/oder Rastverbindungseinrichtung der Kniegelenkendoprothesenvorrichtung zum Verrasten oder Einschnappen der zusammenwirkenden Kupplungselemente beim Erreichen der Zwischenstellung der Kniegelenkendoprothesenvorrichtung auf einfache Weise realisiert werden.

Vorteilhaft ist es, wenn die Rückstelleinrichtung mindestens ein Rückstellelement umfasst und wenn das mindestens eine Rückstellelement dem mindestens einen ersten und/oder zweiten Kupplungselement zugeordnet ist. Insbesondere kann jedem Kupplungselement ein Rückstellelement zugeordnet sein. Auf diese Weise lässt sich insbesondere eine Kopplung zwischen der Tibiakomponente und der Meniskuskomponente auf einfache Weise und für einen Operateur haptisch spürbar erreichen.

Günstigerweise umfasst das mindestens eine erste und/oder zweite Kuopplungselement das mindestens eine Rückstellelement. Diese Ausgestaltung ermöglicht insbesondere einen kompakten Aufbau der Kniegelenkendoprothesenvorrichtung.

Um eine definierte Kopplung zwischen der Tibiakomponente und der Meniskuskomponente zu ermöglichen, ist es günstig, wenn jedem der mindestens einen ersten und/oder zweiten Kupplungselemente jeweils ein Rückstellelement zugeordnet ist.

Günstigerweise ist das mindestens eine Rückstellelement in Form eines Federelements ausgebildet. So lässt sich insbesondere eine Kraft, die aufgebracht werden muss, um die Meniskuskomponente und die Tibiakomponente relativ zueinander von der Trennstellung in die Zwischenstellung zu überführen, einfach und in definierter Weise vorgeben.

Vorteilhaft ist es, wenn der Kopplungsvorsprung mindestens ein Kupplungsglied umfasst, wenn die Kopplungsaufnahme mindestens eine Kupplungsgliedaufnahme umfasst und wenn in der Kopplungsstellung das mindestens eine Kupplungsglied in die mindestens eine Kupplungsgliedaufnahme eingreift. Insbesondere kann die Kniegelenkendoprothesenvorrichtung derart ausgebildet sein, dass das mindestens eine Kupplungsglied und die mindestens eine Kupplungsgliedaufnahme in der Zwischenstellung außer Eingriff stehen. Das mindestens eine Kupplungsglied und die mindestens eine Kupplungsgliedaufnahme können insbesondere zur Sicherung der Meniskuskomponente und der Tibiakomponente in der Kopplungsstellung dienen. Ferner kann durch entsprechende Ausgestaltung bei einer relativ zur Tibiakomponente bewegbar angeordneten Meniskuskomponente eine Bewegung in definierter Weise eingeschränkt werden.

Auf einfache Weise lässt sich die Kniegelenkendoprothesenvorrichtung ausbilden, wenn das mindestens eine Kupplungsglied vom mindestens einen Kupplungselement abstehend angeordnet oder ausgebildet ist. Insbesondere kann es quer zur Anlageebene vom mindestens einen Kupplungselement abstehend angeordnet oder ausgebildet sein.

Günstig ist es, wenn das mindestens eine Kupplungsglied in einer Richtung quer, insbesondere senkrecht, zur Anlageebene vom mindestens einen Kupplungselement weg weisend absteht. Insbesondere kann es von der Anlageebene weg weisend abstehend angeordnet oder ausgebildet sein. Die Kupplungsgliedaufnahme kann insbesondere auch die Kopplungsausnehmung bilden und das mindestens eine Kupplungsglied den mindestens einen Teil des Kopplungsvorsprungs, die in der Kopplungsstellung in Eingriff stehen, aufnehmen.

Vorteilhaft ist es, wenn die Kupplungsgliedaufnahme in Form einer in Richtung auf die Anlageebene hin weisend geöffnete Kupplungsgliedaufnahmenut ausgebildet ist. Eine solche Ausgestaltung ermöglicht es insbesondere, die Kupplungsgliedaufnahme in Form eines Führungselements auszubilden, in welchem das mindestens eine Kopplungsglied in definierter Weise bei einer Relativbewegung zwischen der Tibiakomponente und der Meniskuskomponente geführt ist. Beispielsweise kann die Kupplungsgliedaufnahmenut geradlinig oder gekrümmt ausgebildet sein.

Günstig ist es, wenn der Kopplungsvorsprung mindestens einen ersten Abschnitt, welcher sich direkt von der Oberseite der Tibiakomponente weg erstreckt, und einen sich an den ersten Abschnitt anschließenden zweiten Abschnitt umfasst und wenn eine maximale Ausdehnung des ersten Abschnitts in einer Ebene parallel zur Anlageebene kleiner ist als eine maximale Ausdehnung des zweiten Abschnitts in einer Ebene parallel zur Anlageebene. Durch diese Ausgestaltung kann insbesondere auf einfache Weise eine Hinterschneidung am Kopplungsvorsprung ausgebildet werden, und zwar im Bereich des ersten Abschnitts. Die Kopplungsaufnahme kann korrespondierend ausgebildet sein und Bereiche aufweisen, die in der Zwischenstellung und/oder in der Kopplungsstellung zwischen den zweiten Abschnitt und der Oberseite der Tibiakomponente, also im Bereich des ersten Abschnitts, eingreifen.

Um insbesondere eine Kniegelenkendoprothese ausbilden zu können, die eine möglichst natürliche Bewegung eines Kniegelenks nachbilden kann, ist es günstig, wenn die die Meniskuskomponente und die Tibiakomponente in der Kopplungsstellung relativ zueinander bewegbar sind. Insbesondere können sie verdrehbar und/oder verschiebbar aneinander gehalten sein.

Gemäß einer weiteren bevorzugten Ausführungsform der Erfindung kann vorgesehen sein, dass die Kopplungseinrichtung eine Drehlagereinrichtung zum drehbaren Lagern der Meniskuskomponente und der Tibiakomponente relativ zueinander in der Kopplungsstellung bildet. Diese Ausgestaltung ermöglicht eine insbesondere kompakte Ausbildung der Kniegelenkendoprothesenvorrichtung, da die Kopplungseinrichtung gleichzeitig auch die Funktion der Drehlagereinrichtung übernehmen kann.

Ein Aufbau der Kniegelenkendoprothesenvorrichtung lässt sich insbesondere dadurch besonders kompakt realisieren, dass die Drehlagereinrichtung das mindestens eine erste Kopplungselement und das mindestens eine zweite Kopplungselement umfasst.

Vorteilhaft ist es, wenn das mindestens eine erste Kopplungselement und das mindestens eine zweite Kopplungselement rotationssymmetrisch oder im Wesentlichen rotationssymmetrisch ausgebildet sind und eine Rotationsachse definieren, bezüglich derer die Meniskuskomponente und die Tibiakomponente in der Kopplungsstellung relativ zueinander verdrehbar aneinander gelagert sind. Eine Rotationsachse lässt sich auf einfache Weise durch eine rotationssymmetrische oder im Wesentlichen rotationssymmetrische Ausbildung des mindestens einen ersten oder des mindestens einen zweiten Kopplungselements realisieren.

Vorzugsweise verläuft die Rotationsachse quer, insbesondere senkrecht, zur Anlageebene. Auf diese Weise kann insbesondere erreicht werden, dass die Unterseite der Meniskuskomponente bei einer Rotation relativ zur Tibiakomponente flächig an der Oberseite der Tibiakomponente anliegen kann, und zwar unabhängig davon, welche Rotationsstellung die Meniskuskomponente und die Tibiakomponente relativ zueinander einnehmen.

Vorteilhaft ist es, wenn die die mindestens einen ersten und/oder zweiten Kupplungselemente von der Rotationsachse weg oder auf die Rotationsachse hin verschieb- und/oder verschwenkbar angeordnet oder ausgebildet sind. Derartige Kupplungselemente ermöglichen es auf einfache Weise, eine Kopplungsvorsprungbreite beim Überführen der Kniegelenkendoprothesenvorrichtung von der Trennstellung in die Zwischenstellung und umgekehrt temporär zu verkleinern.

Vorteilhafterweise ist die Kupplungsgliedaufnahmenut die Rotationsachse konzentrisch umgebend angeordnet oder ausgebildet. Dies ermöglicht es insbesondere, die Kupplungsgliedaufnahmenut als ein Element zum Begrenzen einer Rotation der Meniskuskomponente und der Tibiakomponente relativ zueinander auszubilden, also insbesondere als Teil einer Rotationsbegrenzungseinrichtung. Ferner kann durch die Kupplungsgliedaufnahmenut auch wie bereits beschrieben eine Relativbewegung der Tibiakomponente und der Meniskuskomponente in definierter Weise geführt werden. Beispielsweise kann so die Kupplungsgliedaufnahmenut einen Teil der Drehlagereinrichtung bilden.

Günstig ist es, wenn zwischen der Oberseite der Tibiakomponente und dem zweiten Abschnitt im Bereich des ersten Abschnitts eine parallel zur Anlageebene geöffnete Hinterschneidung ausgebildet ist. Insbesondere kann diese in Form einer mindestens abschnittsweise, insbesondere vollständig, umlaufenden, von der Rotationsachse weg weisenden Nut ausgebildet sein. Durch die Hinterschneidung kann insbesondere eine Bewegung der Meniskuskomponente und der Tibiakomponente voneinander weg und auch aufeinander zu in definierter Weise begrenzt werden.

Günstig ist es, wenn die Kopplungsaufnahme mindestens eine parallel zur Unterseite der Meniskuskomponente und in Richtung auf die Rotationsachse hin weisend geöffnete Hinterschneidung aufweist. Insbesondere kann diese in Form einer mindestens abschnittsweise, insbesondere vollständig, umlaufenden, auf die Rotationsachse hin weisenden Nut ausgebildet sein. Diese Ausgestaltung ermöglicht es insbesondere, den zweiten Abschnitt des Kopplungsvorsprungs in diese Hinterschneidung einzuführen beim Übergang von der Trennstellung in die Zwischenstellung. Insbesondere kann die Hinterschneidung derart ausgebildet sein, dass Kopplungselemente am Kopplungsvorsprung in die Hinterschneidung eingreifen, nachdem der Kopplungsvorsprung durch die Einführöffnung durchgetreten ist.

Gemäß einer weiteren bevorzugten Ausführungsform der Erfindung kann vorgesehen sein, dass die Kniegelenkendoprothesenvorrichtung eine Rotationsbegrenzungseinrichtung umfasst zum Begrenzen einer Verdrehung der Meniskuskomponente und der Tibiakomponente relativ zueinander in der Kopplungsstellung. Durch die Rotationsbegrenzungseinrichtung kann also insbesondere eine Relativbewegung zwischen der Meniskuskomponente und der Tibiakomponente in definierter Weise eingeschränkt werden. Eine solche Verdrehung definiert bei einem Kniegelenk insbesondere eine Rotation um eine Beinlängsachse des Patienten.

Günstig ist es, wenn die Rotationsbegrenzungseinrichtung mindestens eine an der Tibiakomponente angeordnete oder ausgebildete erste Anschlagfläche und mindestens eine mit dieser zusammenwirkende, an der Meniskuskomponente angeordnete oder ausgebildete zweite Anschlagfläche umfasst und wenn die mindestens eine erste Anschlagfläche und die mindestens eine zweite Anschlagfläche in einer aus einer Grundstellung um einen maximalen Rotationswinkel ausgelenkten Rotationsstellung aneinander anliegen. Mit einer solchen Rotationsbegrenzungseinrichtung lässt sich auf einfache Weise eine Verdrehung der Meniskuskomponente und der Tibiakomponente bezogen auf die Rotationsachse relativ zueinander begrenzen. Insbesondere kann die Rotationsbegrenzungseinrichtung eine Verdrehbewegung in zueinander entgegengesetzten Drehrichtungen durch entsprechende Anschlagflächen an der Meniskuskomponente und der Tibiakomponente in definierter Weise begrenzen. Beispielsweise kann ein Verdrehwinkel aus einer unausgelenkten Stellung etwa 30 Grad sowohl in die eine als auch in die andere Richtung betragen.

Auf einfache und kompakte Weise lässt sich die Kniegelenkendoprothesenvorrichtung ausbilden, wenn die mindestens eine erste Anschlagfläche und die mindestens eine zweite Anschlagfläche quer, insbesondere senkrecht, zur Anlageebene verlaufen. Insbesondere können so Anschlagkräfte aufgenommen werden, ohne dass dies eine Relativbewegung der Tibiakomponente und der Meniskuskomponente in einer Richtung senkrecht zur Anlageebene zur Folge hat.

Vorteilhaft ist es, wenn die Meniskuskomponente eine Meniskuskomponentenoberseite aufweist, welche mediale und laterale Meniskusgelenkflächen aufweist. Diese Meniskusgelenkflächen können insbesondere mit korrespondierenden Femurgelenkflächen an einer Femurkomponente einer Kniegelenkendoprothese zusammenwirken.

Die eingangs gestellte Aufgabe wird ferner bei einer Kniegelenkendoprothese der eingangs beschriebenen Art erfindungsgemäß dadurch gelöst, dass die Kniegelenkendoprothesenvorrichtung in Form einer der oben beschrieben Kniegelenkendoprothesenvorrichtungen ausgebildet ist.

Die Kniegelenkendoprothese weist dann die bereits oben im Zusammenhang mit bevorzugten Ausführungsformen von Kniegelenkendoprothesenvorrichtungen beschriebenen Vorteile auf.

Vorteilhaft ist es, wenn die Femurkomponente in Richtung auf die Meniskuskomponente weisende mediale und laterale Femurgelenkflächen umfasst, welche in einer Implantationsstellung der Kniegelenkendoprothese an medialen und lateralen Meniskusgelenkflächen der Meniskuskomponente anliegen und mit diesen bei einer Relativbewegung der Femurkomponente und der Meniskuskomponente zusammenwirken. Auf diese Weise lässt sich insbesondere eine Bewegung der Femurkomponente und der Meniskuskomponente relativ zueinander realisieren, die einer Relativbewegung einer natürlichen Tibia relativ zum natürlichen Femur sehr nahe kommt.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Gesamtansicht eines Ausführungsbeispiels einer Kniegelenkendoprothese;
- Figur 2:: eine posteriore Ansicht eines Teils des in Figur 1 dargestellten Ausführungsbeispiels einer Kniegelenkendoprothese in Form einer Kniegelenkendoprothesenvorrichtung;
- Figur 3:: eine Explosionsdarstellung des in Figur 2 dargestellten Ausführungsbeispiels einer Kniegelenkendoprothesenvorrichtung in der Trennstellung;
- Figur 4:: eine Schnittansicht längs Linie 4-4 in Figur 2;
- Figur 5:: eine Ansicht der Tibiakomponente des Ausführungsbeispiels der Kniegelenkendoprothesenvorrichtung aus Figur 2 in Richtung des Pfeils A in Figur 3;
- Figur 6:: eine schematische perspektivische Ansicht der Tibiakomponente aus Figur 5;
- Figur 7:: eine Ansicht der Meniskuskomponente des Ausführungsbeispiels der Kniegelenkendoprothesenvorrichtung aus Figur 2 in Richtung des Pfeils B in Figur 3;
- Figur 8:: eine Schnittansicht der Meniskuskomponente des Ausführungsbeispiels der Kniegelenkendoprothesenvorrichtung aus Figur 2 längs Linie 4-4;
- Figur 9:: eine schematische, teilweise geschnittene Seitenansicht eines weiteren Ausführungsbeispiels einer Kniegelenkendoprothesenvorrichtung in der Trennstellung;
- Figur 10:: eine schematische, teilweise geschnittene Ansicht der Kniegelenkendoprothesenvorrichtung aus Figur 9 in der Zwischenstellung;
- Figur 11:: eine Ansicht der Kniegelenkendoprothesenvorrichtung ähnlich Figur 10 in der Kopplungsstellung;
- Figur 12:: eine perspektivische Ansicht der Tibiakomponente des Ausführungsbeispiels der Kniegelenkendoprothesenvorrichtung aus Figur 9;
- Figur 13:: eine teilweise durchbrochene perspektivische Gesamtansicht eines weiteren Ausführungsbeispiels einer Kniegelenkendoprothesenvorrichtung in der Kopplungsstellung;
- Figur 14:: eine perspektivische Ansicht des Ausführungsbeispiels einer Kniegelenkendoprothesenvorrichtung aus Figur 13 in der Trennstellung;
- Figur 15:: eine Ansicht des Ausführungsbeispiels einer Kniegelenkendoprothesenvorrichtung in der Trennstellung in Richtung des Pfeils C in Figur 14;
- Figur 16:: eine perspektivische anteriore Gesamtansicht des Ausführungsbeispiels der Kniegelenkendoprothesenvorrichtung aus Figur 13 in der Kopplungsstellung; und
- Figur 17:: eine Schnittansicht längs Linie 17-17 in Figur 16.

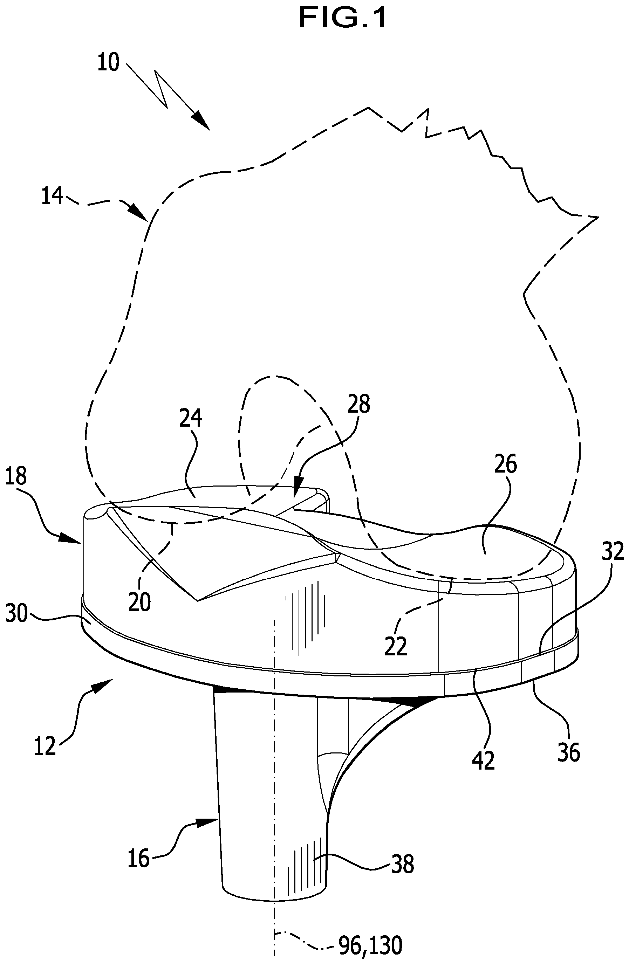

In Figur 1 ist schematisch ein erstes Ausführungsbeispiel einer Kniegelenkendoprothese 10 dargestellt. Sie umfasst ein erstes Ausführungsbeispiel einer Kniegelenkendoprothesenvorrichtung 12 und ein erstes Ausführungsbeispiel einer in Figur 1 schematisch gestrichelt eingezeichneten Femurkomponente 14.

Die Kniegelenkendoprothesenvorrichtung 12 umfasst eine Tibiakomponente 16 zum Verankern an einer Tibia eines Patienten sowie eine Meniskuskomponente 18.

Die Femurkomponente 14 ist ausgebildet zum Festlegen an einem Femur eines Patienten. Sie umfasst mediale und laterale Femurgelenkflächen 20 und 22, die in Richtung auf die Meniskuskomponente 18 hin weisen und in einer Implantationsstellung an korrespondierenden medialen und lateralen Meniskusgelenkflächen 24 beziehungsweise 26 der Meniskuskomponente 18 anliegen und mit diesen bei einer Relativbewegung der Femurkomponente 14 und Meniskuskomponente 18 zusammenwirken.

Die medialen und lateralen Meniskusgelenkflächen 24 und 26 sind auf einer in Richtung auf die Femurkomponente 14 hin weisenden Meniskuskomponentenoberseite 28 angeordnet beziehungsweise ausgebildet.

Die Tibiakomponente 16 umfasst eine Tibiaplatte 30, welche eine Oberseite 32 aufweist, die eben ist und eine Anlageebene 34 definiert. Von einer Unterseite 36 der Tibiaplatte 30 ist ein Schaft 38 abstehend angeordnet, welcher optional mit für den Patienten geeigneten Schaftverlängerungen 40 auf eine erforderliche Länge verlängert werden kann zum Einführen in einen Markkanal der Tibia des Patienten, so dass die Tibiakomponente 16 an der Tibia des Patienten sicher festlegbar ist.

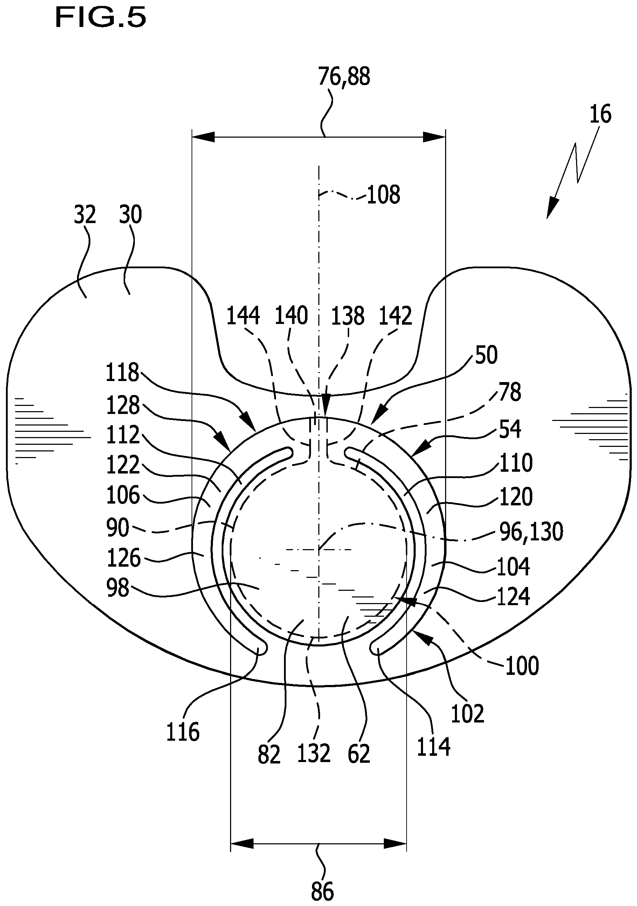

Die Tibiaplatte 30 ist in einer Draufsicht, wie beispielhaft in Figur 5 dargestellt, im Wesentlichen nierenförmig geformt.

Die Tibiakomponente 16 ist aus einem metallischen Werkstoff ausgebildet, beispielsweise aus einem Implantatstahl oder Titan.

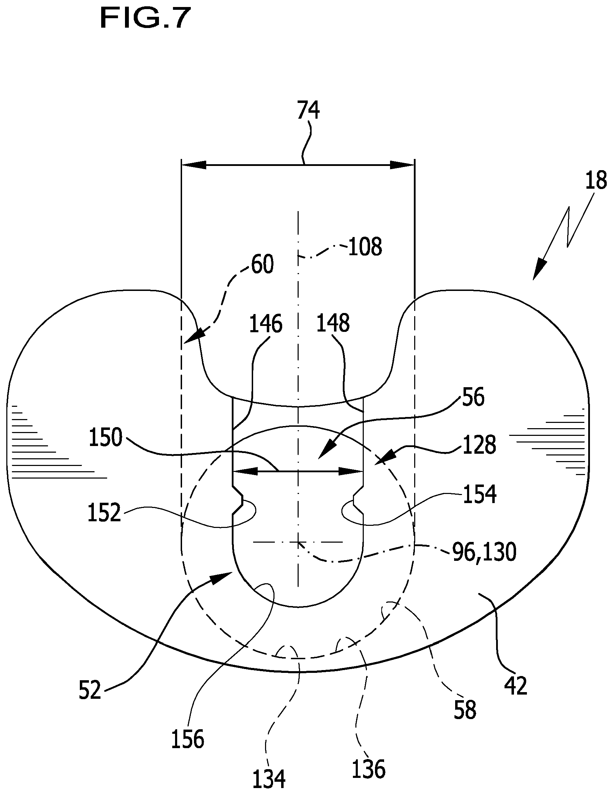

Die Meniskuskomponente weist eine ebene Unterseite 42 auf, die in einer Kopplungsstellung der Kniegelenkendoprothesenvorrichtung 12, in der die Tibiakomponente 16 und die Meniskuskomponente 18 miteinander gekoppelt sind, an der Oberseite 32 anliegt. Die Unterseite 42 weist in einer Draufsicht, wie sie schematisch in Figur 7 dargestellt ist, eine identische Form auf wie die Oberseite 32, sodass die Meniskuskomponente 18 in einer unausgelenkten Grundstellung die Tibiaplatte 30 vollständig bedeckt.

Zum Koppeln der Tibiakomponente 16 und der Meniskuskomponente 18 umfasst die Kniegelenkendoprothesenvorrichtung 12 eine Kopplungseinrichtung 44.

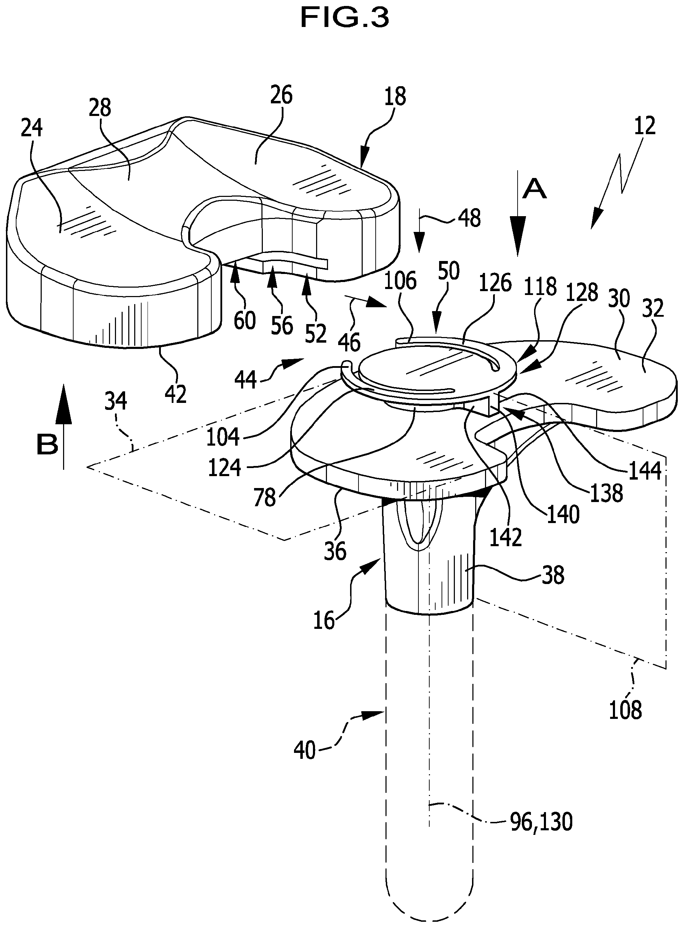

Die Kniegelenkendoprothesenvorrichtung 12 ist derart ausgebildet, dass sie von einer Trennstellung, in der die Tibiakomponente 16 und die Meniskuskomponente 18 vollständig voneinander getrennt sind, wie schematisch in Figur 3 dargestellt, in die Kopplungsstellung überführbar ist durch eine Relativbewegung der Tibiakomponente 16 und der Meniskuskomponente 18. Insbesondere ist die Kopplungseinrichtung 44 derart ausgebildet, dass die Tibiakomponente 16 und die Meniskuskomponente 18 beim Überführen der Kniegelenkendoprothesenvorrichtung 12 von der Trennstellung in die Kopplungsstellung in einer Richtung parallel zur Anlageebene 34 relativ zueinander zunächst von der Trennstellung in eine Zwischenstellung überführt werden können. Diese Bewegung ist in Figur 3 symbolisiert durch den Pfeil 46, welcher parallel zur Anlageebene 34 ausgerichtet ist.

Von der Zwischenstellung kann die Kniegelenkendoprothesenvorrichtung 12 in die Kopplungsstellung überführt werden, und zwar durch eine Bewegung in einer Richtung quer, symbolisiert durch den Pfeil 48, zur Anlageebene 34, sodass die Meniskuskomponente 18 und die Tibiakomponente 16 aufeinander zu bewegt werden, bis die Unterseite 42 an der Oberseite 32 anliegt.

Um diese Funktion der Kopplungseinrichtung 44 zu ermöglichen, umfasst diese ein erstes Kopplungselement 50 an der Tibiakomponente 16 und ein korrespondierendes zweites Kopplungselement 52 an der Meniskuskomponente 18. Die ersten und zweiten Kopplungselemente 50 und 52 stehen in der Trennstellung außer Eingriff und in der Kopplungsstellung in Eingriff.

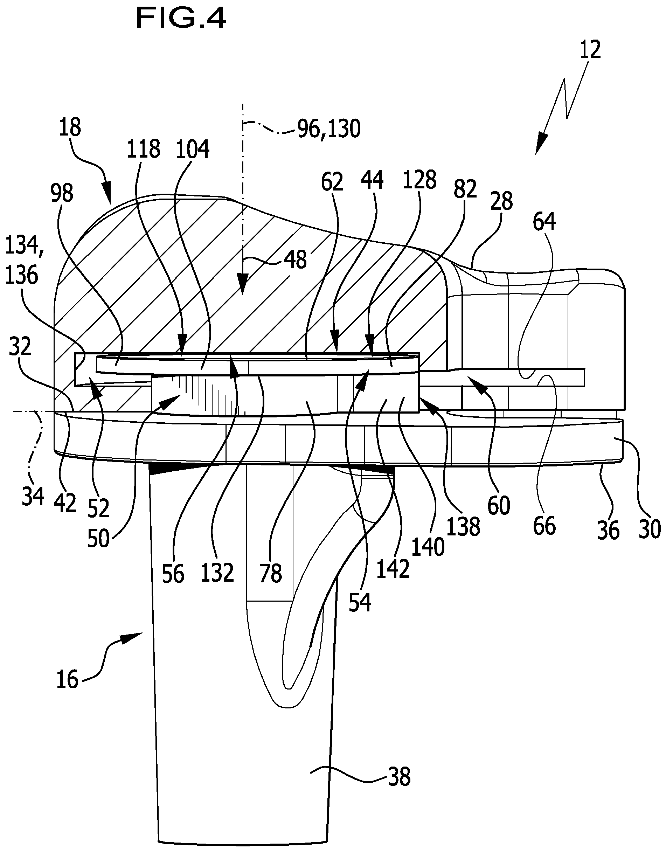

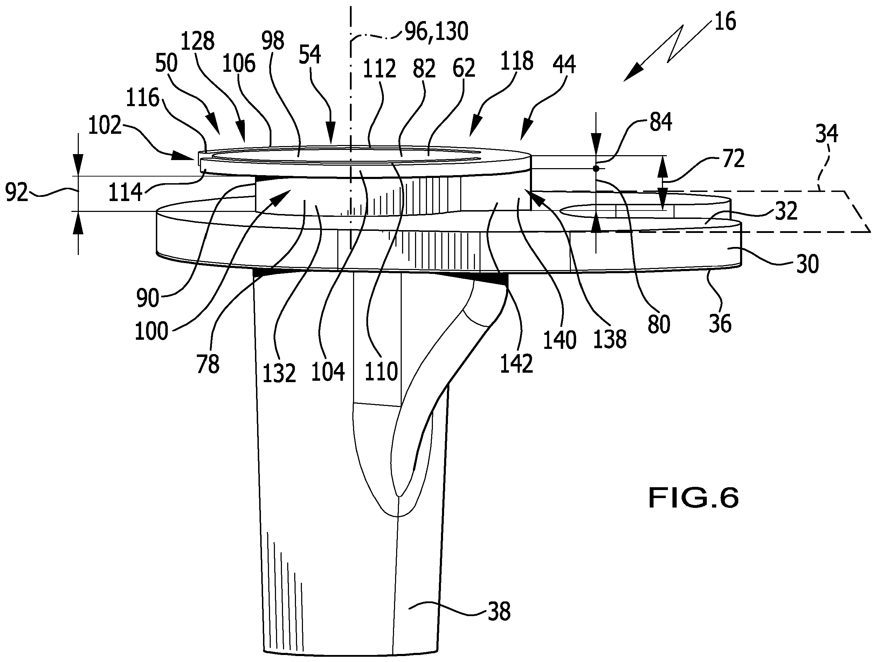

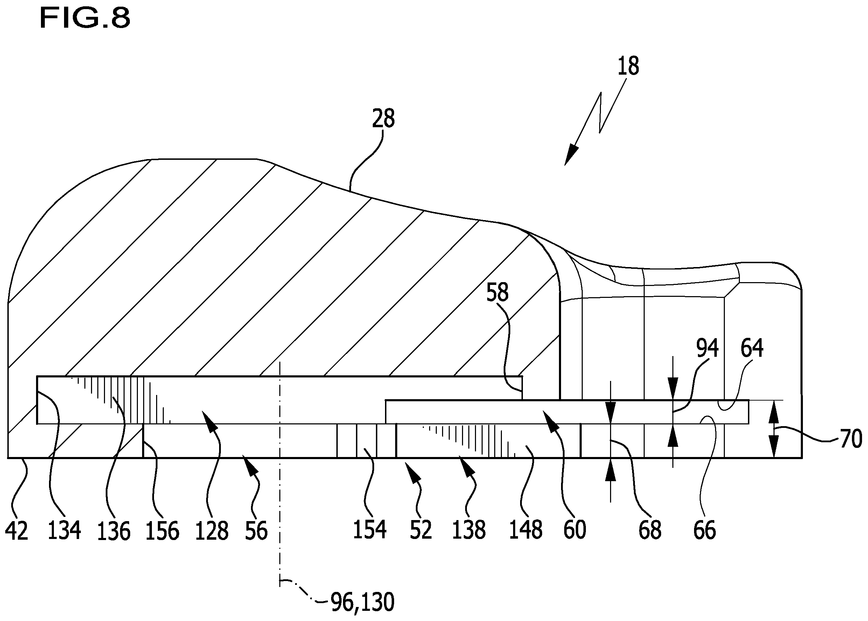

Bei dem in den Figuren 1 bis 8 dargestellten Ausführungsbeispiel der Kniegelenkendoprothesenvorrichtung 12 ist das erste Kopplungselement 50 in Form eines Kopplungsvorsprungs 54 ausgebildet. Das zweite Kopplungselement 52 ist in Form einer Kopplungsaufnahme 56 ausgebildet.

Die Kopplungsaufnahme 56 an der Meniskuskomponente 18 umfasst ferner eine Kopplungsausnehmung 58 in Form einer Ausnehmung.

Die Kopplungsaufnahme 56 ist ausgebildet, um den Kopplungsvorsprung 54 in der Zwischenstellung aufzunehmen. Mit anderen Worten können die Tibiakomponente 16 und die Meniskuskomponente 18 ausgehend von der Trennstellung in die Zwischenstellung überführt werden, wobei dann der Kopplungsvorsprung 54 in die Kopplungsaufnahme 56 eingeführt wird.

Die Kopplungsausnehmung 58 nimmt in der Kopplungsstellung einen Teil des Kopplungsvorsprungs 54 auf. Um dies zu ermöglichen, und zwar bei einer Bewegung der Meniskuskomponente 18 und der Tibiakomponente 16 aufeinander zu, ist die Kopplungsausnehmung 58 in einer Richtung senkrecht zur Anlageebene 34 geöffnet. Mit anderen Worten bildet die Kopplungsausnehmung 58 eine von der Anlageebene 34 zurückgesetzte Vertiefung oder Hinterschneidung der Kopplungsaufnahme 56.

Der Kopplungsvorsprung 54 umfasst einen zylindrischen Kopplungskörper 78, welcher eine Höhe 80 bezogen auf die Oberseite 32 aufweist und einen ersten Abschnitt 100 definiert. Der Kopplungsvorsprung 54 umfasst ferner eine Kopplungsscheibe 82, die auf dem Kopplungskörper 78 angeordnet ist, eine Dicke 84 aufweist und einen zweiten Abschnitt 102 definiert. Ein Durchmesser 86 des Kopplungskörpers 78 und somit eine maximale Ausdehnung desselben in einer Ebene parallel zur Anlageebene 34 ist kleiner als ein Durchmesser 88 des zweiten Abschnitts 102. Zwischen der Kopplungsscheibe 82 und der Tibiaplatte ist somit eine bezogen auf eine Längsachse 96 des Kopplungskörpers 78, welche senkrecht zur Anlageebene 34 verläuft, umlaufende und von der Längsachse 96 weg weisend geöffnete Nut 90 ausgebildet, deren Breite 92 der Höhe 80 entspricht.

Die Kopplungsscheibe 82 des Kopplungsvorsprungs 54 weist eine von der Anlageebene 34 weg weisende Kopplungsvorsprungoberseite 62 auf.

Um den Kopplungsvorsprung 54 in die Kopplungsaufnahme 56 einführen zu können, weist die Meniskuskomponente 18 eine Einführöffnung 60 auf. Durch diese hindurch kann der Kopplungsvorsprung 54 in die Kopplungsaufnahme 56 eingeführt werden.

Die Einführöffnung 60 der Meniskuskomponente 18 definiert eine obere Begrenzungsfläche 64 und eine untere Begrenzungsfläche 66. Beide Begrenzungsflächen 64 und 66 verlaufen parallel zur Unterseite 42 der Meniskuskomponente 18.

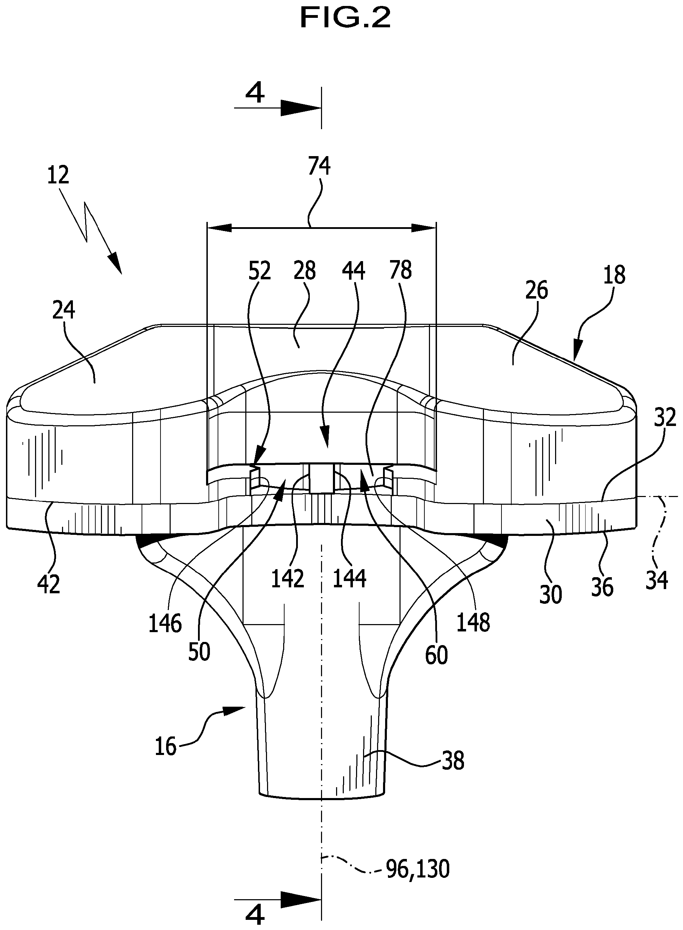

Ein Abstand 68 der unteren Begrenzungsfläche 66 von der Unterseite 42 ist kleiner als ein Abstand 70 der oberen Begrenzungsfläche 64 von der Unterseite 42. Ferner ist ein Abstand 72 der Kopplungsvorsprungoberseite 62 von der Anlageebene 34 größer als der Abstand 68. Eine Breite 94 der Einführöffnung 60 parallel zur Längsachse 96 entspricht einer Differenz der Abstände 70 und 68 und ist unwesentlich größer als die Dicke 84 des zweiten Abschnitts 102, so dass die Kopplungsscheibe 82 durch die von posterior gesehen, wie schematisch in Figur 2 dargestellt, schlitzförmige Einführöffnung 60 parallel zur Anlageebene 34 eingeschoben werden kann.

Die beschriebene Beziehung zwischen dem Abstand 68 und dem Abstand 72 hat zur Folge, dass die Unterseite 42 beim Überführen der Kniegelenkendoprothesenvorrichtung 12 von der Trennstellung in die Zwischenstellung beabstandet ist, und zwar um einen Abstand, welcher einer Differenz zwischen den Abständen 72 und 68 entspricht.

Die Einführöffnung 60 definiert eine Einführbreite 74. Der Kopplungsvorsprung 54 definiert im Bereich der Kopplungsscheibe 82 eine Kopplungsvorsprungbreite 76. Bei dem in den Figuren 1 bis 8 dargestellten Ausführungsbeispiel der Kniegelenkendoprothesenvorrichtung 12 ist die Kopplungsvorsprungbreite 76 größer als die Einführbreite 74.

Um den Kopplungsvorsprung 54 durch die Einführöffnung 60 hindurchführen zu können, muss die Kopplungsvorsprungbreite 76 temporär verringert werden. Dies wird dadurch erreicht, dass der zweite Abschnitt 102 zwei bewegliche Kupplungselemente 104 und 106 umfasst, die, ebenso wie der Kopplungsvorsprung 54, spiegelsymmetrisch zu einer die Längsachse 96 enthaltenden Spiegelebene 108 ausgebildet sind, die in anterior-posteriorer Richtung verläuft.

Die Kupplungselemente 104 und 106 sind ausgebildet durch jeweils einen halbkreisförmigen, konzentrisch zur Längsachse 96 verlaufenden Schlitz 110 beziehungsweise 112 im zweiten Abschnitt 102. Freie Enden 114 und 116 der Kupplungselemente 104 und 106 sind etwas voneinander beabstandet und anteriorseitig am Kopplungsvorsprung 54 positioniert.

Die Kupplungselemente 104 und 106 sind parallel zur Anlageebene 34 bewegbar angeordnet beziehungsweise ausgebildet.

Die Kupplungselemente 104 und 106 sind entgegen der Wirkung einer Rückstelleinrichtung 118 aus einer Kupplungselementgrundstellung, in welcher die Rückstelleinrichtung 118 keine Rückstellkraft auf die Kupplungselemente 104 und 106 ausübt, in eine Kupplungselementauslenkstellung auslenkbar. Die Kupplungselemente 104 und 106 sind insbesondere mit ihren freien Enden 114 und 116 aufeinander zu bewegbar, wenn sie durch die schlitzförmige Einführöffnung 60 hindurchgeführt werden. Dadurch verringert sich die Kopplungsvorsprungbreite 76 temporär etwas.

Sobald der zweite Abschnitt 102 in die Kopplungsaufnahme 56 eingeführt ist, können die Kupplungselemente 104 und 106 wieder in ihre ursprüngliche Kupplungselementgrundstellung ausschwenken. Der Kopplungsvorsprung 54 ist auf diese Weise in die Kopplungsaufnahme 56 eingeschnappt.

Die Rückstelleinrichtung 118 umfasst zwei Rückstellelemente 120 und 122, die jeweils einem Kupplungselement 104 beziehungsweise 106 zugeordnet sind. Bei dem in den Figuren 1 bis 8 dargestellten Ausführungsbeispiel der Kniegelenkendoprothesenvorrichtung 12 umfasst jedes Kupplungselement 104, 106 jeweils ein Rückstellelement 120 beziehungsweise 122.

Die Rückstellelemente 120 und 122 sind jeweils in Form eines Federelements 124 beziehungsweise 126 ausgebildet.

Die Meniskuskomponente 18 und die Tibiakomponente 16 sind in der Kopplungsstellung relativ zueinander bewegbar. Sie können relativ zueinander um die Längsachse 96 verdreht werden. Dies wird ermöglicht durch die Kopplungseinrichtung 44, die eine Drehlagereinrichtung 128 zum drehbaren Lagern der Meniskuskomponente 18 und der Tibiakomponente 16 relativ zueinander in der Kopplungsstellung umfasst beziehungsweise bildet.

Die Drehlagereinrichtung 128 umfasst die beiden Kopplungselemente 50 und 52.

Das erste Kopplungselement 50 und das zweite Kopplungselement 52 sind jeweils rotationssymmetrisch beziehungsweise im Wesentlichen rotationssymmetrisch ausgebildet und definieren eine Rotationsachse 130, die mit der Längsachse 96 zusammenfällt. Die Meniskuskomponente 18 und die Tibiakomponente 16 sind in der Kopplungsstellung relativ zueinander verdrehbar aneinander gelagert bezüglich der Rotationsachse 130.

Die Rotationsachse 130 verläuft senkrecht zur Anlageebene 34.

Wie bereits beschrieben sind die Kupplungselemente 104 und 106 auf die Rotationsachse 130 hin verschwenkbar ausgebildet beziehungsweise angeordnet.

Die zwischen der Oberseite 32 der Tibiakomponente 16 und dem zweiten Abschnitt 102 im Bereich des ersten Abschnitts 100 ausgebildete Nut 90 bildet eine parallel zur Anlageebene 34 geöffnete Hinterschneidung 132.

Die Kopplungsaufnahme 56 an der Meniskuskomponente 18 weist eine parallel zur Unterseite 42 derselben und in Richtung auf die Längsachse 96 beziehungsweise die Rotationsachse 130 hin weisend geöffnete Hinterschneidung 134 auf. Sie ist abschnittsweise in Form einer auf die Rotationsachse 130 hin weisenden Nut 136 ausgebildet.

Das beschriebene Ausführungsbeispiel der Kniegelenkendoprothesenvorrichtung 12 umfasst ferner eine Rotationsbegrenzungseinrichtung 139 zum Begrenzen einer Verdrehung der Meniskuskomponente 18 und der Tibiakomponente 16 relativ zueinander in der Kopplungsstellung.

Die Rotationsbegrenzungseinrichtung 138 umfasst einen Anschlag 140, der in Form eines vom ersten Abschnitt 100 in posteriorer Richtung weisend abstehenden Vorsprungs ausgebildet ist, welcher zwei voneinander weg weisende Anschlagflächen 142 und 144 definiert.

Mit den Anschlagflächen 142 und 144 wirken aufeinander zu weisende Anschlagflächen 146 und 148 der Meniskuskomponente 18 zusammen, die die Einführöffnung 60 zwischen der unteren Begrenzungsfläche 66 und der Unterseite 42 begrenzen. Ein Abstand 150 zwischen den Anschlagflächen 146 und 148 ist etwas größer als der Durchmesser 86 des ersten Abschnitts 100, sodass dieser beim Koppeln der Meniskuskomponente 18 und der Tibiakomponente 16 miteinander durch die Einführöffnung 60 in die Kopplungsaufnahme 56 eingeführt werden kann.

Die Anschlagfläche 142 begrenzt mit der Anschlagfläche 146 eine Bewegung der Meniskuskomponente 18 relativ zur Tibiakomponente 16 in Draufsicht auf die Oberseite 32 gesehen im Gegenuhrzeigersinn. Entsprechend begrenzen die zusammenwirkenden Anschlagflächen 144 und 148 eine Verdrehbewegung der Meniskuskomponente 18 relativ zur Tibiakomponente 16 um die Rotationsachse 130 im Uhrzeigersinn.

Die beiden wie beschrieben maximal ausgelenkten Rotationsstellungen, in denen die Anschlagflächen 142 und 146 einerseits beziehungsweise die Anschlagflächen 144 und 148 andrerseits aneinander anliegen, begrenzen einen maximalen Rotationswinkel.

Die Anschlagflächen 142, 144 und 146, 148 verlaufen senkrecht zur Anlageebene 34.

An der Meniskuskomponente 18 sind von den Anschlagflächen 146 und 148 vorstehend jeweils kleine Nocken 152 und 154 ausgebildet, die den Abstand 150 zwischen den Anschlagflächen 146 und 148 etwas verringern.

Beim Einführen des Kopplungsvorsprungs 54 durch die Einführöffnung 60 in die Kupplungsaufnahme 56 hinein, muss der erste Abschnitt 100 zwischen den Nocken 152 und 154 durchgeschoben werden. Dies führt zu einer temporären Verformung der aus einem Kunststoff, insbesondere Polyethylen mit ultrahohem Molekulargewicht (UHMWPE), ausgebildeten Meniskuskomponente 18, was als klickendes Geräusch vernehmbar ist. Der erste Abschnitt 100 ist in der Kopplungsstellung dann zwischen den Nocken 152 und 154 sowie einem in Richtung auf die Rotationsachse 130 weisenden, konkaven Rand 156 gesichert.

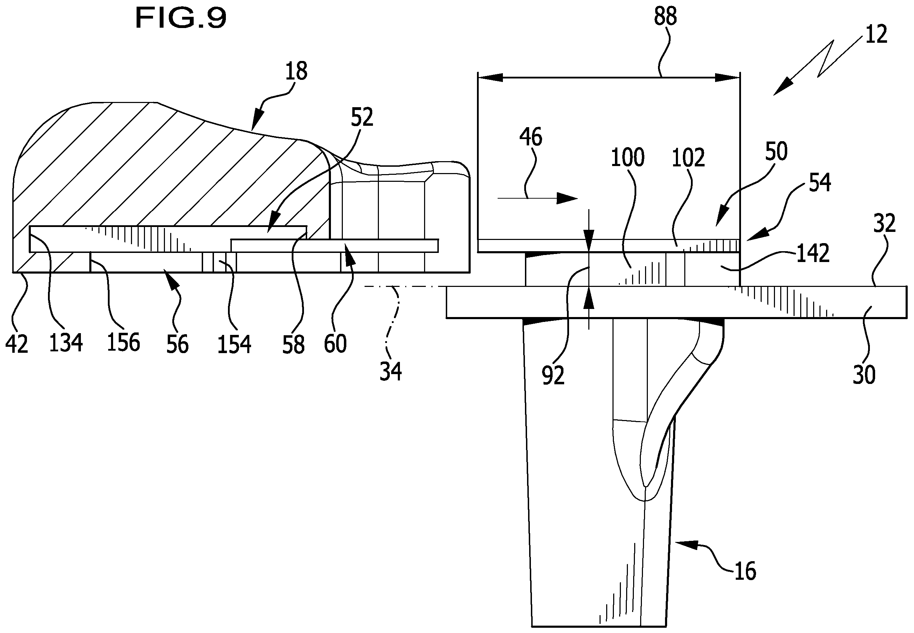

Ein weiteres Ausführungsbespiel einer Kniegelenkendoprothesenvorrichtung 12 ist beispielhaft in den Figuren 9 bis 12 dargestellt. Es stimmt in seinem Aufbau mit den in den Figuren 1 bis 8 dargestellten und oben beschriebenen Ausführungsbeispiel der Kniegelenkendoprothesenvorrichtung 12 im Wesentlichen überein. Der Übersichtlichkeit wegen sind daher identische oder ähnliche Komponenten bei dem in den Figuren 9 bis 12 dargestellten Ausführungsbeispiel der Kniegelenkendoprothesenvorrichtung 12 mit denselben Bezugszeichen versehen wie bei dem in den Figuren 1 bis 8 dargestellten Ausführungsbeispiel der Kniegelenkendoprothesenvorrichtung 12.

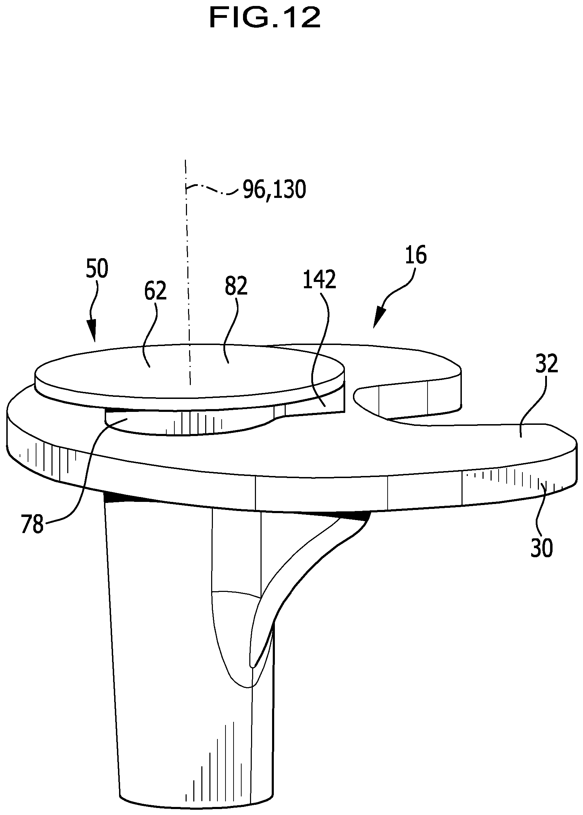

Anders als beim Ausführungsbeispiel der Figuren 1 bis 8 sind beim Ausführungsbeispiel der Figuren 9 bis 12 keine Kupplungselemente 104, 106 vorgesehen. Der zweite Abschnitt 102 ist, wie in Figur 12 gut zu erkennen, in Form einer kreisförmigen Kopplungsscheibe 82 ausgebildet.

Damit der zweite Abschnitt 102 durch die Einführöffnung 60 zwischen die obere Begrenzungsfläche 64 und die untere Begrenzungsfläche 66 eingeführt werden kann, ist die Einführbreite 74 an der Meniskuskomponente 18 etwas größer als der Durchmesser 88 der Kopplungsscheibe 82.

Zum Koppeln der Tibiakomponente 16 und der Meniskuskomponente 18 des in den Figuren 9 bis 12 dargestellten Ausführungsbeispiels der Kniegelenkendoprothesenvorrichtung 12 wird die Meniskuskomponente 18 mit ihrer Unterseite 42 etwas beabstandet von der Oberseite 32 parallel zur Anlageebene 34 verschoben und der zweite Abschnitt 102 zwischen die Begrenzungsflächen 64 und 66 der Einführöffnung 60 eingeführt. Dies bedeutet, dass die Meniskuskomponente 18 von anterior her kommend in posteriorer Richtung auf die Tibiakomponente 16 aufgeschoben wird. Sobald der erste Abschnitt 100 die beiden Nocken 152 und 154 passiert hat, ist ein Klickgeräusch vernehmbar. Der Kopplungsvorsprung 54 ist dann wie oben beschrieben in der Kopplungsaufnahme 56 in der Zwischenstellung gesichert.

Aus dieser Zwischenstellung kann durch eine Bewegung der Meniskuskomponente 18 in Richtung auf die Oberseite 32 der Tibiaplatte 30 hin der zweite Abschnitt 102, also ein Teil des Kopplungsvorsprungs 54, in die Kopplungsausnehmung 48 eintauchen. Die Meniskuskomponente 18 und die Tibiakomponente 16 können nun, soweit dies die Rotationsbegrenzungseinrichtung 138 zulässt, relativ zueinander um die Rotationsachse 130 verdreht werden.

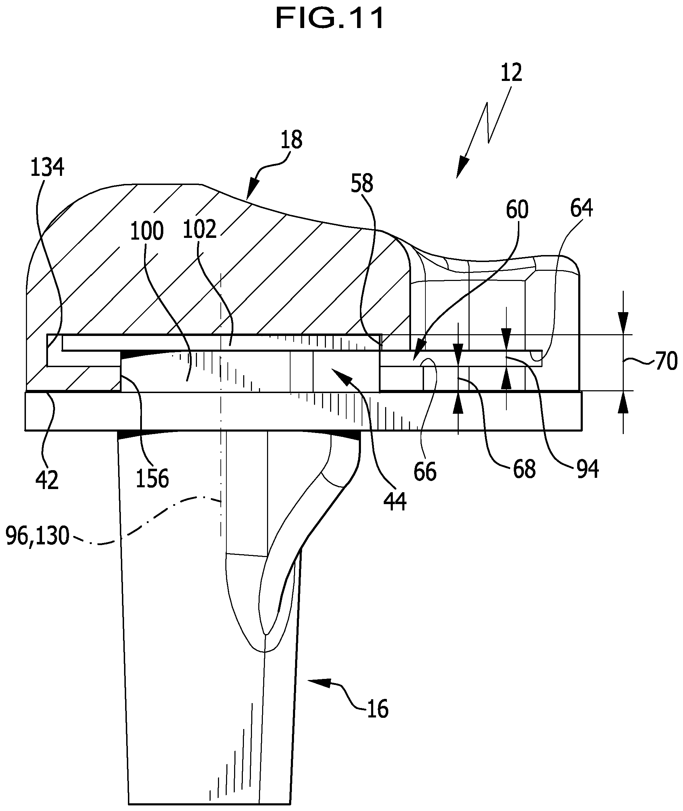

Durch das Eingreifen der Kopplungsscheibe 82 in die Kopplungsausnehmung 58 ist die Meniskuskomponente 18 an der Tibiakomponente 16 gesichert. Eine Verschiebung der Meniskuskomponente 18 relativ zur Tibiakomponente 16 parallel zur Anlageebene 34 ist in dieser, in Figur 11 schematisch dargestellten Kopplungsstellung nicht mehr oder nur sehr eingeschränkt möglich.

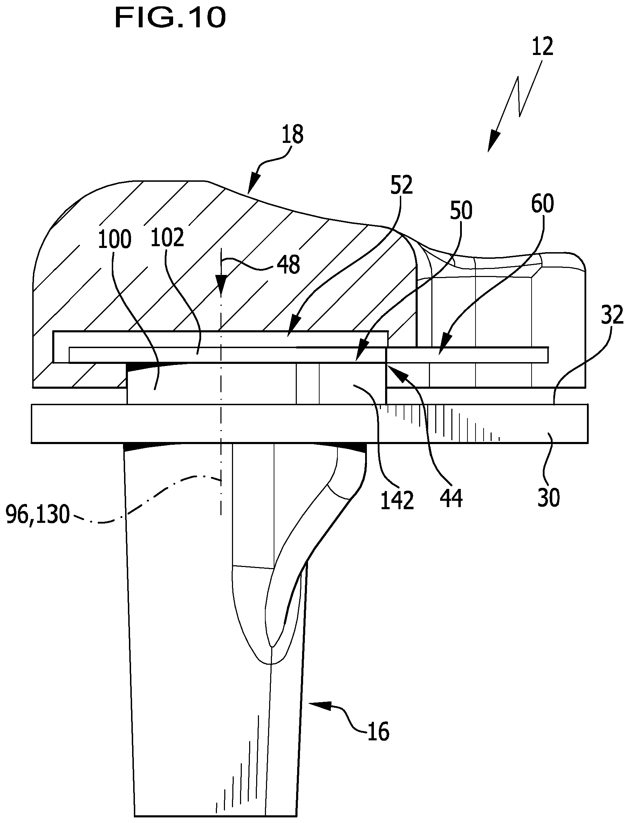

Zum Entfernen der Meniskuskomponente 18 von der Tibiakomponente 16 muss in umgekehrter Reihenfolge zunächst die Meniskuskomponente 18 bezogen auf die Oberseite 32 angehoben werden, um die Kniegelenkendoprothesenvorrichtung 12 in die Zwischenstellung zu überführen. Aus dieser schematisch in Figur 10 dargestellten Zwischenstellung kann dann der zweite Abschnitt 102 wieder durch die Einführöffnung 60 im Bereich zwischen den Begrenzungsflächen 64 und 66 hindurch geführt werden, bis die Kniegelenkendoprothesenvorrichtung 12 wieder die schematisch in Figur 9 dargestellte Trennstellung einnimmt.

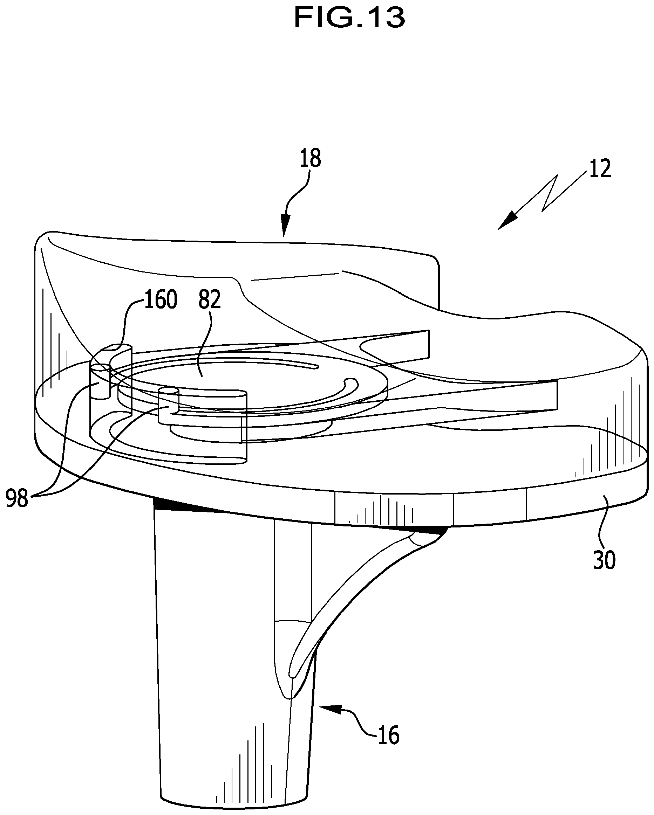

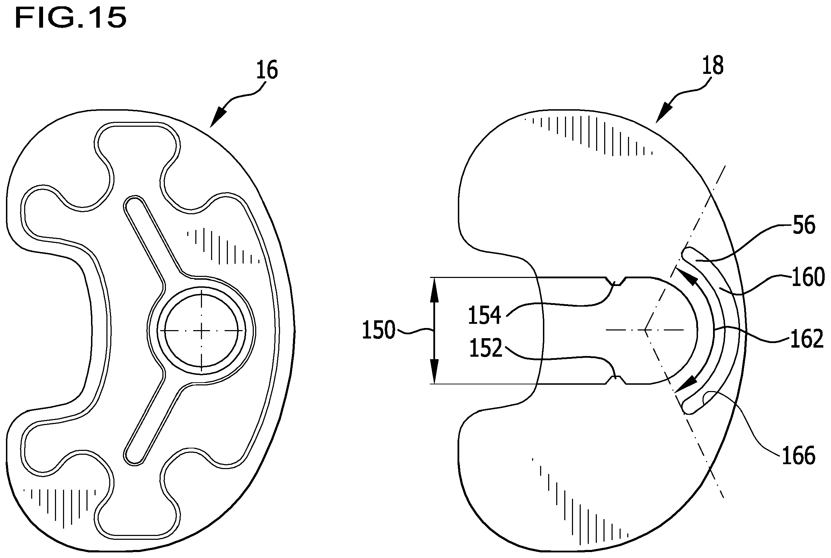

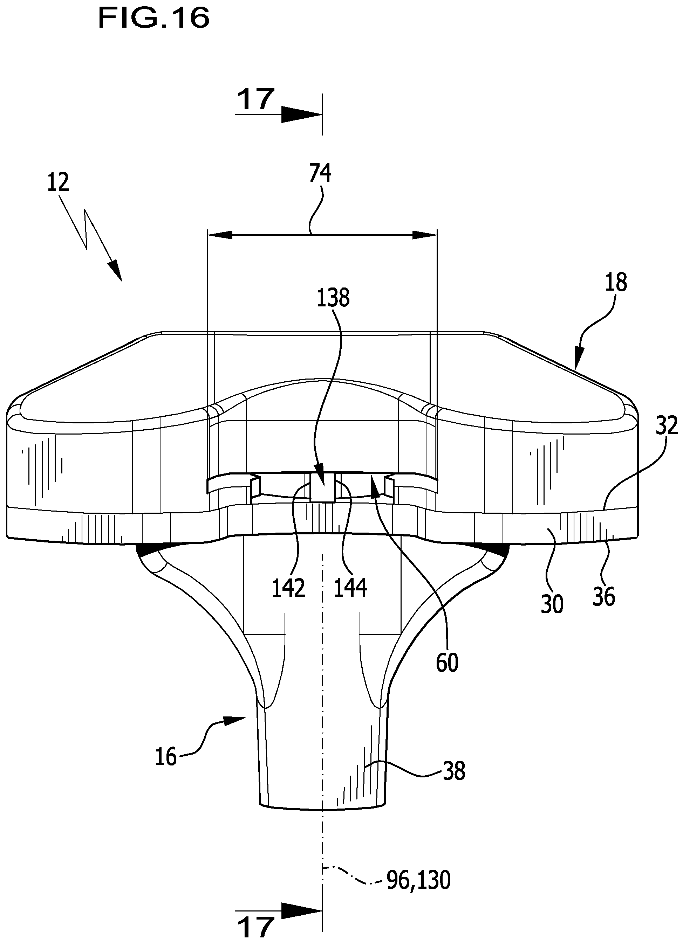

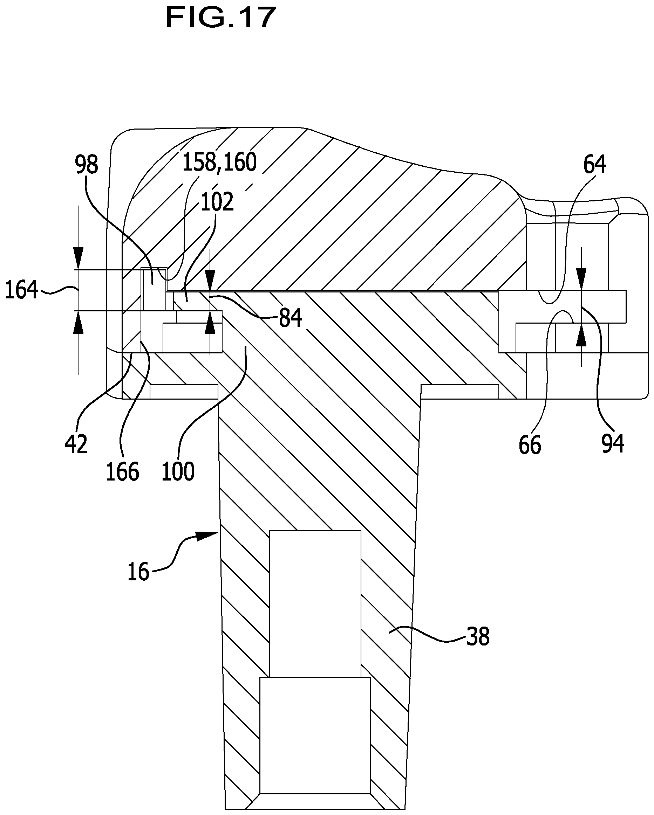

Ein weiteres Ausführungsbeispiel einer Kniegelenkendoprothesenvorrichtung 12 ist schematisch in den Figuren 13 bis 17 dargestellt. Es stimmt in seinem Aufbau weitgehend mit dem im Zusammenhang mit den Figuren 1 bis 8 beschriebenen Ausführungsbeispiel der Kniegelenkendoprothesenvorrichtung 12 überein. Identische Komponenten der Kniegelenkendoprothesenvorrichtung 12 der Figuren 13 bis 17 sind daher mit denselben Bezugszeichen wie bei den beiden oben beschriebenen Ausführungsbeispielen bezeichnet.

Anders als beim Ausführungsbeispiel der Kniegelenkendoprothesenvorrichtung 12 der Figuren 1 bis 8 umfasst der Kopplungsvorsprung 54 zwei Kupplungsglieder 98. Diese stehen in Form kurzer zylindrischer Vorsprünge von den freien Enden 114 und 116 der Kupplungselemente 104 und 106 parallel zur Längsachse 96 ausgerichtet und von der Oberseite 32 der Tibiaplatte 30 weg weisend ab.

An der Meniskuskomponente 18 ist eine konzentrisch zur Längsachse 96 und in Richtung auf die Oberseite 32 hin weisend geöffnete Nut 158 ausgebildet, die eine Kupplungsgliedaufnahme 160 bildet. In diese Nut 158, die sich symmetrisch zur Spiegelebene 108 über einen Winkelbereich 162 von etwa 120° erstreckt, greifen die beiden Kupplungsglieder 98 in der Kopplungsstellung ein. Die Kupplungsgliedaufnahme 160 bildet somit auch die Kopplungsausnehmung 58.

Eine Gesamthöhe 164 der Kupplungsglieder 98 parallel zur Längsachse 96, die auch die Dicke 84 umfasst, erfordert, dass die Breite 94 der Einführöffnung 60 zwischen den Begrenzungsflächen 64 und 66 etwas größer ist als die Gesamthöhe 164. Damit ist auch die Breite 94 beim Ausführungsbeispiel der Kniegelenkendoprothesenvorrichtung 12 der Figuren 13 bis 17 größer als die Breite 94 beim Ausführungsbeispiel der Kniegelenkendoprothesenvorrichtung 12 der Figuren 1 bis 8, und zwar um die Länge der Kupplungsglieder 98 gemessen ab der Kopplungsvorsprungoberseite 62.

Zur Ausbildung der Nut 158 wird die Meniskuskomponente 18 ausgehend von der Unterseite 42 mit einem Fräswerkzeug bearbeitet. Auf diese Weise wird eine zur Nut 158 korrespondierende bogenförmige Durchbrechung 166 in der Meniskuskomponente 18 präpariert.

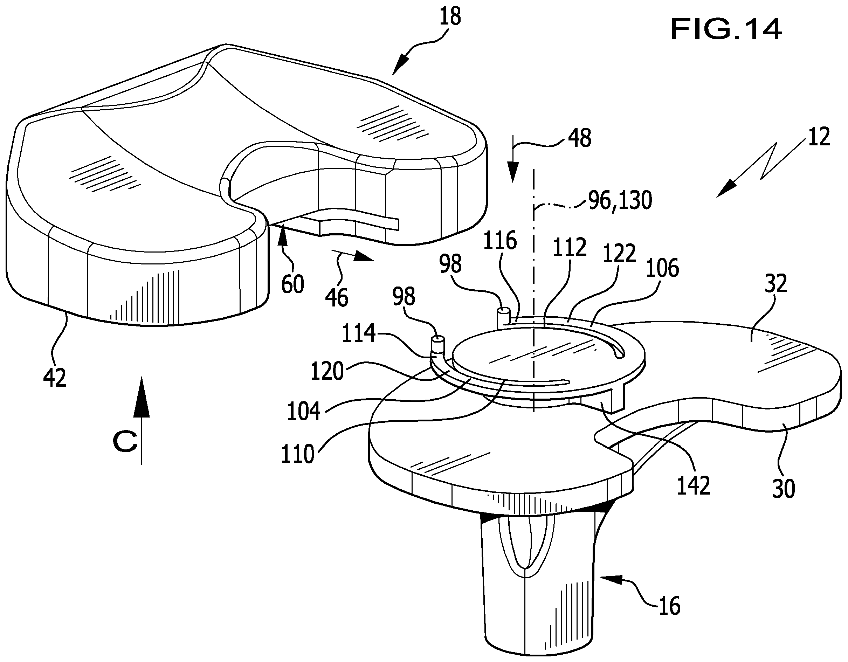

Die Meniskuskomponente 18 und die Tibiakomponente 16 werden ausgehend von der Trennstellung, die schematisch in Figur 14 dargestellt ist, durch Verschieben derselben parallel zur Anlageebene 34 miteinander in Eingriff gebracht. Die freien Enden 114 und 116 mit den Kupplungsgliedern 98 werden durch die Einführöffnung 60 zwischen den Begrenzungsflächen 64 und 66 eingeführt.

Die Einführbreite 74 ist etwas kleiner als der Durchmesser 88 des zweiten Abschnitts 102, so dass beim Einführen des Kopplungsvorsprungs 54 durch die Einführöffnung 60 die Kopplungselemente 104 und 106 etwas in Richtung auf die Längsachse 96 hin verschwenkt werden.

Sobald der erste Abschnitt 100 die beiden Nocken 152 und 154 an der Meniskuskomponente 18 hintergreift, nimmt die Kniegelenkendoprothesenvorrichtung 12 die Zwischenstellung ein.

Durch Bewegung der Meniskuskomponente 18 in Richtung auf die Oberseite 32 der Tibiakomponente 16 hin, tauchen die Kupplungsglieder 98 in die Kupplungsgliedaufnahme 160 ein und sichern die Meniskuskomponente 18 an der Tibiakomponente 16. Die Unterseite 42 der Meniskuskomponente 18 liegt dann in der Kopplungsstellung, die schematisch in den Figuren 13, 16 und 17 dargestellt ist, an der Oberseite 32 der Tibiakomponente 16 an.

Die Rotationsbegrenzungseinrichtung 138 der Kniegelenkendoprothesenvorrichtung 12 ist wie die beim Ausführungsbeispiel der Kniegelenkendoprothesenvorrichtung 12 der Figuren 1 bis 8 ausgebildet.

Die in die Kupplungsgliedaufnahme 160 eingreifenden Kupplungsglieder 98 können, je nach Wahl des Winkelbereichs 162, ebenfalls zur Begrenzung einer Verdrehung der Tibiakomponente 16 und der Meniskuskomponente 18 relativ zueinander beitragen. Aufgrund der zur Längsachse 96 konzentrischen Ausbildung der Nut 158 führen die Kupplungsglieder 98 im Zusammenwirken mit der Nut 158 auch die Verdrehbewegung der Tibiakomponente 16 und der Meniskuskomponente 18 relativ zueinander.

Zum Trennen der Meniskuskomponente 18 von der Tibiakomponente 16 muss wiederum die Meniskuskomponente 18, wie auch bei den anderen oben beschriebenen Ausführungsbeispielen, von der Oberseite 32 etwas angehoben werden, so dass die Kupplungsglieder 98 und die Kupplungsgliedaufnahme 160 außer Eingriff stehen. Aus dieser Zwischenstellung können dann die Meniskuskomponente 18 und die Tibiakomponente 16 relativ zueinander parallel zur Anlageebene 34 verschoben und der Kopplungsvorsprung 54 und die Kopplungsaufnahme 56 außer Eingriff gebracht werden.

Die beschriebenen Ausführungsbeispiele von Kniegelenkendoprothesenvorrichtungen 12 ermöglichen eine bandschonende Implantation. Bei Bedarf ist auch auf einfache Weise ein Austausch der Meniskuskomponente 18 möglich.

### Bezugszeichenliste

- 10: Kniegelenkendoprothese
- 12: Kniegelenkendoprothesenvorrichtung
- 14: Femurkomponente
- 16: Tibiakomponente
- 18: Meniskuskomponente
- 20: Femurgelenkfläche
- 22: Femurgelenkfläche
- 24: Meniskusgelenkfläche
- 26: Meniskusgelenkfläche
- 28: Meniskuskomponentenoberseite
- 30: Tibiaplatte
- 32: Oberseite
- 34: Anlageebene
- 36: Unterseite
- 38: Schaft
- 40: Schaftverlängerungen
- 42: Unterseite
- 44: Kopplungseinrichtung
- 46: Pfeil
- 48: Pfeil
- 50: erstes Kopplungselement
- 52: zweites Kopplungselement
- 54: Kopplungsvorsprung
- 56: Kopplungsaufnahme
- 58: Kopplungsausnehmung
- 60: Einführöffnung
- 62: Kopplungsvorsprungoberseite
- 64: obere Begrenzungsfläche
- 66: untere Begrenzungsfläche
- 68: Abstand
- 70: Abstand
- 72: Abstand
- 74: Einführbreite
- 76: Kopplungsvorsprungbreite
- 78: Kopplungskörper
- 80: Höhe
- 82: Kopplungsscheibe
- 84: Dicke
- 86: Durchmesser
- 88: Durchmesser
- 90: Nut
- 92: Breite
- 94: Breite
- 96: Längsachse
- 98: Kopplungsglied
- 100: erster Abschnitt
- 102: zweiter Abschnitt
- 104: Kupplungselement
- 106: Kupplungselement
- 108: Spiegelebene
- 110: Schlitz
- 112: Schlitz
- 114: Ende
- 116: Ende
- 118: Rückstelleinrichtung
- 120: Rückstellelement
- 122: Rückstellelement
- 124: Federelement
- 126: Federelement
- 128: Drehlagereinrichtung
- 130: Rotationsachse
- 132: Hinterschneidung
- 134: Hinterschneidung
- 136: Nut
- 138: Rotationsbegrenzungseinrichtung
- 140: Anschlag
- 142: Anschlagfläche
- 144: Anschlagfläche
- 146: Anschlagfläche
- 148: Anschlagfläche
- 150: Abstand
- 152: Nocken
- 154: Nocken
- 156: Rand
- 158: Nut
- 160: Kupplungsgliedaufnahme
- 162: Winkelbereich
- 164: Gesamthöhe
- 166: Durchbrechung

## Patentansprüche

1. Kniegelenkendoprothesenvorrichtung (12) umfassend eine Tibiakomponente (16) zum Verankern an einer Tibia und eine Meniskuskomponente (18), wobei die Tibiakomponente (16) und die Meniskuskomponente (18) in einer Kopplungsstellung miteinander gekoppelt und in einer Trennstellung vollständig voneinander getrennt sind, welche Tibiakomponente (16) eine Anlageebene (34) definierende Oberseite (32) aufweist, an welcher eine Unterseite (36) der Meniskuskomponente (18) in der Kopplungsstellung anliegt, wobei die Kniegelenkendoprothesenvorrichtung (12) von der Trennstellung durch eine Relativbewegung der Tibiakomponente (16) und der Meniskuskomponente (18) in die Kopplungsstellung überführbar ist, **dadurch gekennzeichnet, dass** die Kniegelenkendoprothesenvorrichtung (12) eine Kopplungseinrichtung (44) umfasst zum Koppeln der Tibiakomponente (16) und der Meniskuskomponente (18) in der Kopplungsstellung, dass die Kopplungseinrichtung (44) derart ausgebildet ist, dass die Tibiakomponente (16) und die Meniskuskomponente (18) beim Überführen der Kniegelenkendoprothesenvorrichtung (12) von der Trennstellung in die Kopplungsstellung in einer Richtung (46) parallel zur Anlageebene (34) relativ zueinander von der Trennstellung in eine Zwischenstellung und von der Zwischenstellung in einer Richtung (48) quer, insbesondere senkrecht, zur Anlageebene (34) und aufeinander zu in die Kopplungsstellung verschiebbar sind.

2. Kniegelenkendoprothesenvorrichtung nach Anspruch 1, **dadurch gekennzeichnet, dass** die Kopplungseinrichtung (44) mindestens ein erstes Kopplungselement (50) und mindestens ein zweites Kopplungselement (52) umfasst, dass das mindestens eine erste Kopplungselement (50) an der Tibiakomponente (16) oder an der Meniskuskomponente (18) angeordnet oder ausgebildet ist, dass das mindestens eine zweite Kopplungselement (52) an der Meniskuskomponente (18) oder an der Tibiakomponente (16) angeordnet oder ausgebildet ist, dass das mindestens eine erste Kopplungselement (50) und das mindestens eine zweite Kopplungselement (52) in der Trennstellung außer Eingriff stehen und dass das mindestens eine erste Kopplungselement (50) und das mindestens eine zweite Kopplungselement (52) in der Kopplungsstellung in Eingriff stehen.

3. Kniegelenkendoprothesenvorrichtung nach Anspruch 2, **dadurch gekennzeichnet, dass** das mindestens eine erste Kopplungselement (50) in Form eines Kopplungsvorsprungs (54) ausgebildet ist, dass das mindestens eine zweite Kopplungselement (52) in Form einer Kopplungsaufnahme (56) mit einer Kopplungsausnehmung (58) ausgebildet ist, dass die Kopplungsaufnahme (56) ausgebildet ist zum Aufnehmen des Kopplungsvorsprungs (54) in der Zwischenstellung und dass die Kopplungsausnehmung (58) ausgebildet ist zum Aufnehmen mindestens eines Teils des Kopplungsvorsprungs (54) in der Kopplungsstellung,
wobei insbesondere
a) die Kopplungsausnehmung (58)
- in einer Richtung senkrecht zur Anlageebene (34) geöffnet ist und/oder
- ausschließlich in einer Richtung senkrecht zur Anlageebene (34) geöffnet ist
und/oder
b) die Meniskuskomponente (18) die Kopplungsausnehmung (58) umfasst, die in Richtung auf die Tibiakomponente (16) hin weisend geöffnet ist zum Einführen des mindestens einen Teils des Kopplungsvorsprungs (54) infolge einer Relativbewegung von der Zwischenstellung in die Kopplungsstellung in der Richtung quer, insbesondere senkrecht, zur Anlageebene (34).

4. Kniegelenkendoprothesenvorrichtung nach Anspruch 3, **dadurch gekennzeichnet, dass** die Meniskuskomponente (18) eine Einführöffnung (60) aufweist zum Einführen des Kopplungsvorsprungs (54) in die Kopplungsaufnahme (56) der Meniskuskomponente (18) hinein,
wobei insbesondere der Kopplungsvorsprung (54) eine von der Anlageebene (34) weg weisende Kopplungsvorsprungoberseite (62) aufweist, wobei die Einführöffnung (60) eine obere Begrenzungsfläche (64) und eine untere Begrenzungsfläche (66) definiert, die parallel zur Unterseite (42) der Meniskuskomponente (18) verlaufen, wobei ein Abstand (68) der unteren Begrenzungsfläche (66) von der Unterseite (42) der Meniskuskomponente (18) kleiner ist als ein Abstand (70) der oberen Begrenzungsfläche (64) von der Unterseite (42) der Meniskuskomponente (18) und wobei der Abstand (68) der unteren Begrenzungsfläche (66) von der Unterseite (42) der Meniskuskomponente kleiner ist als ein Abstand (72) der Kopplungsvorsprungoberseite (62) von der Anlageebene (34).

5. Kniegelenkendoprothesenvorrichtung nach Anspruch 4, **dadurch gekennzeichnet, dass** die Einführöffnung (60) eine Einführbreite (74) definiert, dass der Kopplungsvorsprung (54) eine Kopplungsvorsprungbreite (76) definiert und dass die Kopplungsvorsprungbreite (76) mindestens der Einführbreite (74) entspricht,
wobei insbesondere die Kopplungsvorsprungbreite (76) größer als die Einführbreite (74) ist.

6. Kniegelenkendoprothesenvorrichtung nach Anspruch 5, **dadurch gekennzeichnet, dass** am Kopplungsvorsprung (54) und/oder an der Einführöffnung (60) mindestens erste und/oder zweite bewegliche Kupplungselemente (104, 106) angeordnet oder ausgebildet sind und dass die Kupplungselemente (104, 106) beim Übergang von der Trennstellung in die Zwischenstellung bewegbar sind zum temporären Verkleinern der Kopplungsvorsprungbreite (76) und/oder zum Vergrößern der Einführbreite (74),
wobei insbesondere die mindestens einen ersten und/oder zweiten Kupplungselemente (104, 106) parallel zur Anlageebene (34) bewegbar angeordnet oder ausgebildet sind.

7. Kniegelenkendoprothesenvorrichtung nach Anspruch 6, **dadurch gekennzeichnet, dass** die mindestens einen ersten und/oder zweiten Kupplungselemente (104, 106) entgegen der Wirkung einer Rückstelleinrichtung (118) aus einer Kupplungselementgrundstellung, in welcher die Rückstelleinrichtung (118) keine Rückstellkraft auf die mindestens einen ersten und/oder zweiten Kupplungselemente (104, 106) ausübt, in eine Kopplungselementauslenkstellung auslenkbar sind.

8. Kniegelenkendoprothesenvorrichtung nach Anspruch 7, **dadurch gekennzeichnet, dass** die Rückstelleinrichtung (118) mindestens ein Rückstellelement (120, 122) umfasst und dass das mindestens eine Rückstellelement (120, 122) dem mindestens einen ersten und/oder zweiten Kupplungselement (104, 106) zugeordnet ist,
wobei insbesondere
a) das mindestens ein erste und/oder zweite Kupplungselement (104,
106) das mindestens eine Rückstellelement (120, 122) umfasst und/oder
b) jedem der mindestens einen ersten und/oder zweiten Kupplungselemente (104, 106) jeweils ein Rückstellelement (102, 122) zugeordnet ist
und/oder
c) das mindestens eine Rückstellelement (120, 122) in Form eines Federelements (124, 126) ausgebildet ist.

9. Kniegelenkendoprothesenvorrichtung nach einem der Ansprüche 3 bis 8, **dadurch gekennzeichnet, dass** der Kopplungsvorsprung (54) mindestens ein Kupplungsglied (98) umfasst, dass die Kopplungsaufnahme (56) mindestens eine Kupplungsgliedaufnahme (160) umfasst und dass in der Kopplungsstellung das mindestens eine Kupplungsglied (98) in die mindestens eine Kupplungsgliedaufnahme eingreift,
wobei insbesondere
a) das mindestens eine Kupplungsglied (98) vom mindestens einen Kupplungselement (104, 106) abstehend angeordnet oder ausgebildet ist
und/oder
b) das mindestens eine Kupplungsglied (98) in einer Richtung quer, insbesondere senkrecht, zur Anlageebene (34) vom mindestens einen Kupplungselement (104, 106) weg weisend absteht, insbesondere von der Anlageebene (34) weg weisend,
und/oder
c) die Kupplungsgliedaufnahme (160) in Form einer in Richtung auf die Anlageebene (34) hin weisend geöffnete Kupplungsgliedaufnahmenut (158) ausgebildet ist.

10. Kniegelenkendoprothesenvorrichtung nach einem der Ansprüche 3 bis 9, **dadurch gekennzeichnet, dass**
a) der Kopplungsvorsprung (54) mindestens einen ersten Abschnitt (100), welcher sich direkt von der Oberseite (32) der Tibiakomponente (16) weg erstreckt, und einen sich an den ersten Abschnitt (100) anschließenden zweiten Abschnitt (102) umfasst und dass eine maximale Ausdehnung des ersten Abschnitts (100) in einer Ebene parallel zur Anlageebene (34) kleiner ist als eine maximale Ausdehnung des zweiten Abschnitts (102) in einer Ebene parallel zur Anlageebene (34),
wobei insbesondere zwischen der Oberseite (32) der Tibiakomponente (16) und dem zweiten Abschnitt (102) im Bereich des ersten Abschnitts (100) eine parallel zur Anlageebene (34) geöffnete Hinterschneidung (132) ausgebildet ist, insbesondere in Form einer mindestens abschnittsweise, insbesondere vollständig, umlaufenden, von der Rotationsachse (130) weg weisenden Nut (90),
und/oder
b) die Kopplungsaufnahme (56) mindestens eine parallel zur Unterseite (42) der Meniskuskomponente (18) und in Richtung auf die Rotationsachse (130) hin weisend geöffnete Hinterschneidung (134) aufweist, insbesondere in Form einer mindestens abschnittsweise, insbesondere vollständig, umlaufenden, auf die Rotationsachse (130) hin weisenden Nut (136).

11. Kniegelenkendoprothesenvorrichtung nach einem der voranstehenden Ansprüche, **dadurch gekennzeichnet, dass** die Meniskuskomponente (18) und die Tibiakomponente (16) in der Kopplungsstellung relativ zueinander bewegbar sind, insbesondere verdrehbar und/oder verschiebbar.

12. Kniegelenkendoprothesenvorrichtung nach einem der voranstehenden Ansprüche, **dadurch gekennzeichnet, dass** die Kopplungseinrichtung (44) eine Drehlagereinrichtung (128) zum drehbaren Lagern der Meniskuskomponente (18) und der Tibiakomponente (16) relativ zueinander in der Kopplungsstellung bildet,
wobei insbesondere die Drehlagereinrichtung (128) das mindestens eine erste Kopplungselement (50) und das mindestens eine zweite Kopplungselement (52) umfasst.

13. Kniegelenkendoprothesenvorrichtung nach einem der Ansprüche 2 bis 12, **dadurch gekennzeichnet, dass** das mindestens eine erste Kopplungselement (50) und das mindestens eine zweite Kopplungselement (52) rotationssymmetrisch oder im Wesentlichen rotationssymmetrisch ausgebildet sind und eine Rotationsachse (130) definieren, bezüglich derer die Meniskuskomponente (18) und die Tibiakomponente (16) in der Kopplungsstellung relativ zueinander verdrehbar aneinander gelagert sind, wobei insbesondere
a) die Rotationsachse (130) quer, insbesondere senkrecht, zur Anlageebene (34) verläuft
und/oder
b) die mindestens einen ersten und/oder zweiten Kupplungselemente (104, 106) von der Rotationsachse (130) weg oder auf die Rotationsachse (130) hin verschieb- und/oder verschwenkbar angeordnet oder ausgebildet sind
und/oder
c) die Kupplungsgliedaufnahmenut (158) die Rotationsachse (130) konzentrisch umgebend angeordnet oder ausgebildet ist.

14. Kniegelenkendoprothesenvorrichtung nach einem der voranstehenden Ansprüche, **dadurch gekennzeichnet, dass**
a) die Kniegelenkendoprothesenvorrichtung (12) eine Rotationsbegrenzungseinrichtung (138) umfasst zum Begrenzen einer Verdrehung der Meniskuskomponente (18) und der Tibiakomponente (16) relativ zueinander in der Kopplungsstellung,
wobei insbesondere die Rotationsbegrenzungseinrichtung (138) mindestens eine an der Tibiakomponente (16) angeordnete oder ausgebildete erste Anschlagfläche (142, 144) und mindestens eine mit dieser zusammenwirkende, an der Meniskuskomponente (18) angeordnete oder ausgebildete zweite Anschlagfläche (146, 148) umfasst und wobei die mindestens eine erste Anschlagfläche (142, 144) und die mindestens eine zweite Anschlagfläche (146, 148) in einer aus einer Grundstellung um einen maximalen Rotationswinkel ausgelenkten Rotationsstellung aneinander anliegen,
wobei weiter insbesondere die mindestens eine erste Anschlagfläche (142, 144) und die mindestens eine zweite Anschlagfläche (146, 148) quer, insbesondere senkrecht, zur Anlageebene (34) verlaufen,
und/oder
b) die Meniskuskomponente (18) eine Meniskuskomponentenoberseite (28) aufweist, welche mediale und laterale Meniskusgelenkflächen (24, 28) aufweist.

15. Kniegelenkendoprothese (10) umfassend eine Kniegelenkendoprothesenvorrichtung (12) und eine mit dieser zusammenwirkende Femurkomponente (14), **dadurch gekennzeichnet, dass** die Kniegelenkendoprothesenvorrichtung (12) in Form einer Kniegelenkendoprothesenvorrichtung (12) nach einem der voranstehenden Ansprüche ausgebildet ist,
wobei insbesondere die Femurkomponente (14) in Richtung auf die Meniskuskomponente (18) weisende mediale und laterale Femurgelenkflächen (20, 22) umfasst, welche in einer Implantationsstellung der Kniegelenkendoprothese (10) an medialen und lateralen Meniskusgelenkflächen (24, 26) der Meniskuskomponente (18) anliegen und mit diesen bei einer Relativbewegung der Femurkomponente (14) und der Meniskuskomponente (18) zusammenwirken.

## Claims

1. Knee joint endoprosthesis apparatus (12) comprising a tibial component (16) for anchoring to a tibia, and comprising a meniscal component (18), wherein the tibial component (16) and the meniscal component (18) are coupled to one another in a coupling position and are completely separated from one another in a separating position, which tibial component (16) has a top side (32) defining an abutment plane (34), against which top side (32) a bottom side (36) of the meniscal component (18) abuts in the coupling position, wherein the knee joint endoprosthesis apparatus (12) is transferrable from the separating position into the coupling position by a relative movement of the tibial component (16) and the meniscal component (18), **characterized in that** the knee joint endoprosthesis apparatus (12) comprises a coupling device (44) for coupling the tibial component (16) and the meniscal component (18) in the coupling position, **in that** the coupling device (44) is configured in such a way that the tibial component (16) and the meniscal component (18), upon transferring the knee joint endoprosthesis apparatus (12) from the separating position into the coupling position, are displaceable relative to one another in a direction (46) parallel to the abutment plane (34) from the separating position into an intermediate position and from the intermediate position in a direction (48) transverse, in particular perpendicular, to the abutment plane (34) and toward one another into the coupling position.

2. Knee joint endoprosthesis apparatus in accordance with Claim 1, **characterized in that** the coupling device (44) comprises at least one first coupling element (50) and at least one second coupling element (52), **in that** the at least one first coupling element (50) is arranged or formed on the tibial component (16) or on the meniscal component (18), **in that** the at least one second coupling element (52) is arranged or formed on the meniscal component (18) or on the tibial component (16), **in that** the at least one first coupling element (50) and the at least one second coupling element (52) are out of engagement in the separating position, and **in that** the at least one first coupling element (50) and the at least one second coupling element (52) are in engagement in the coupling position.

3. Knee joint endoprosthesis apparatus in accordance with Claim 2, **characterized in that** the at least one first coupling element (50) is configured in the form of a coupling projection (54), **in that** the at least one second coupling element (52) is configured in the form of a coupling receptacle (56) with a coupling recess (58), **in that** the coupling receptacle (56) is configured to accommodate the coupling projection (54) in the intermediate position, and **in that** the coupling recess (58) is configured to accommodate at least a part of the coupling projection (54) in the coupling position,
wherein, in particular,
a) the coupling recess (58)
- is open in a direction perpendicular to the abutment plane (34)
and/or
- is open exclusively in a direction perpendicular to the abutment plane (34)
and/or
b) the meniscal component (18) comprises the coupling recess (58), which is open facing in the direction toward the tibial component (16) for inserting the at least one part of the coupling projection (54) as the result of a relative movement from the intermediate position into the coupling position in the direction transverse, in particular perpendicular, to the abutment plane (34).

4. Knee joint endoprosthesis apparatus in accordance with Claim 3, **characterized in that** the meniscal component (18) has an insertion opening (60) for inserting the coupling projection (54) into the coupling receptacle (56) of the meniscal component (18),
wherein, in particular, the coupling projection (54) has a coupling projection top side (62) facing away from the abutment plane (34), wherein the insertion opening (60) defines an upper delimiting face (64) and a lower delimiting face (66), which extend in parallel to the bottom side (42) of the meniscal component (18), wherein a distance (68) of the lower delimiting face (66) from the bottom side (42) of the meniscal component (18) is smaller than a distance (70) of the upper delimiting face (64) from the bottom side (42) of the meniscal component (18), and wherein the distance (68) of the lower delimiting face (66) from the bottom side (42) of the meniscal component is smaller than a distance (72) of the coupling projection top side (62) from the abutment plane (34).

5. Knee joint endoprosthesis apparatus in accordance with Claim 4, **characterized in that** the insertion opening (60) defines an insertion width (74), **in that** the coupling projection (54) defines a coupling projection width (76), and **in that** the coupling projection width (76) corresponds to at least the insertion width (74),
wherein, in particular, the coupling projection width (76) is greater than the insertion width (74).

6. Knee joint endoprosthesis apparatus in accordance with Claim 5, **characterized in that** at least first and/or second moveable coupling elements (104, 106) are arranged or formed on the coupling projection (54) and/or on the insertion opening (60), and **in that** the coupling elements (104, 106) upon the transition from the separating position into the intermediate position are moveable for temporarily reducing the coupling projection width (76) and/or for enlarging the insertion width (74),
wherein, in particular, the at least one first and/or second coupling elements (104, 106) are arranged or formed so as to be moveable in parallel to the abutment plane (34).

7. Knee joint endoprosthesis apparatus in accordance with Claim 6, **characterized in that** the at least one first and/or second coupling elements (104, 106) are deflectable against the action of a restoring device (118) from a coupling element base position, in which the restoring device (118) does not exert a restoring force on the at least one first and/or second coupling elements (104, 106), into a coupling element deflected position.

8. Knee joint endoprosthesis apparatus in accordance with Claim 7, **characterized in that** the restoring device (118) comprises at least one restoring element (120, 122), and **in that** the at least one restoring element (120, 122) is associated with the at least one first and/or second coupling element (104, 106),
wherein, in particular,
a) the at least one first and/or second coupling element (104, 106) comprises the at least one restoring element (120, 122)
and/or
b) a respective restoring element (120, 122) is associated with each of the at least one first and/or second coupling elements (104, 106)
and/or
c) the at least one restoring element (120, 122) is configured in the form of a spring element (124, 126).

9. Knee joint endoprosthesis apparatus in accordance with any one of Claims 3 to 8, **characterized in that** the coupling projection (54) comprises at least one coupling member (98), **in that** the coupling receptacle (56) comprises at least one coupling member receptacle (160), and **in that**, in the coupling position, the at least one coupling member (98) engages into the at least one coupling member receptacle, wherein, in particular,
a) the at least one coupling member (98) is arranged or formed projecting from the at least one coupling element (104, 106)
and/or
b) the at least one coupling member (98) projects facing away from the at least one coupling element (104, 106), in particular facing away from the abutment plane (34), in a direction transverse, in particular perpendicular, to the abutment plane (34),
and/or
c) the coupling member receptacle (160) is configured in the form of a coupling member receptacle groove (158) that is open facing in the direction toward the abutment plane (34).

10. Knee joint endoprosthesis apparatus in accordance with any one of Claims 3 to 9, **characterized in that**
a) the coupling projection (54) comprises at least one first portion (100), which extends directly from the top side (32) of the tibial component (16), and a second portion (102) adjoining the first portion (100), and **in that** a maximum extent of the first portion (100) in a plane parallel to the abutment plane (34) is smaller than a maximum extent of the second portion (102) in a plane parallel to the abutment plane (34),
wherein, in particular an undercut (132) that is open in parallel to the abutment plane (34) is formed between the top side (32) of the tibial component (16) and the second portion (102) in the region of the first portion (100), in particular in the form of a groove (90) that is all-round at least in sections, in particular completely, and faces away from the axis of rotation (130),
and/or
b) the coupling receptacle (56) has at least one undercut (134) that is open in parallel to the bottom side (42) of the meniscal component (18) and faces in the direction toward the axis of rotation (130), said undercut (134) being configured, in particular, in the form of a groove (136) that is all-round at least in sections, in particular completely, and faces toward the axis of rotation (130).

11. Knee joint endoprosthesis apparatus in accordance with any one of the preceding Claims, **characterized in that** the meniscal component (18) and the tibial component (16) in the coupling position are moveable, in particular rotatable and/or displaceable, relative to one another.

12. Knee joint endoprosthesis apparatus in accordance with any one of the preceding Claims, **characterized in that** the coupling device (44) forms a rotary bearing device (128) for rotatably bearing the meniscal component (18) and the tibial component (16) relative to one another in the coupling position,
wherein, in particular, the rotatory bearing device (128) comprises the at least one first coupling element (50) and the at least one second coupling element (52).

13. Knee joint endoprosthesis apparatus in accordance with any one of Claims 2 to 12, **characterized in that** the at least one first coupling element (50) and the at least one second coupling element (52) are formed rotationally symmetrical or substantially rotationally symmetrical and define an axis of rotation (130), in relation to which the meniscal component (18) and the tibial component (16) in the coupling position are rotatably mounted relative to one another,
wherein, in particular,
a) the axis of rotation (130) extends transversely, in particular perpendicularly, to the abutment plane (34)
and/or
b) the at least one first and/or second coupling elements (104, 106) are arranged or formed so as to be displaceable and/or pivotable away from the axis of rotation (130) or toward the axis of rotation (130)
and/or
c) the coupling member receptacle groove (158) is arranged or formed concentrically surrounding the axis of rotation (130).

14. Knee joint endoprosthesis apparatus in accordance with any one of preceding Claims, **characterized in that**
a) the knee joint endoprosthesis apparatus (12) comprises a rotation limiting device (138) for limiting a rotation of the meniscal component (18) and the tibial component (16) relative to one another in the coupling position,
wherein, in particular, the rotation limiting device (138) comprises at least one first stop face (142, 144) arranged or formed on the tibial component (16) and at least one second stop face (146, 148) that cooperates therewith and is arranged or formed on the meniscal component (18), and wherein the at least one first stop face (142, 144) and the at least one second stop face (146, 148) abut against one another in a rotation position deflected from a base position by a maximum rotation angle,
wherein, further in particular, the at least one first stop face (142, 144) and the at least one second stop face (146, 148) extend transversely, in particular perpendicularly, to the abutment plane (34),
and/or
b) the meniscal component (18) has a meniscal component top side (28), which has medial and lateral meniscal joint faces (24, 28).

15. Knee joint endoprosthesis (10) comprising a knee joint endoprosthesis apparatus (12) and a femoral component (14) that cooperates therewith, **characterized in that** the knee joint endoprosthesis apparatus (12) is configured in the form of a knee joint endoprosthesis apparatus (12) in accordance with any one of the preceding Claims,
wherein, in particular, the femoral component (14) comprises medial and lateral femoral joint faces (20, 22) facing in the direction toward the meniscal component (18), which femoral joint faces (20, 22) in an implantation position of the knee joint endoprosthesis (10) abut against medial and lateral meniscal joint faces (24, 26) of the meniscal component (18) and cooperate therewith upon a relative movement of the femoral component (14) and the meniscal component (18).

## Revendications

1. Dispositif d'endoprothèse d'articulation du genou (12) comprenant une composante pour tibia (16) pour l'ancrage contre un tibia et une composante pour ménisque (18), dans lequel la composante pour tibia (16) et la composante pour ménisque (18) sont couplées l'une à l'autre dans une position d'accouplement et complètement séparées l'une de l'autre dans une position de séparation, ladite composante pour tibia (16) présentant un côté supérieur (32) définissant un plan d'application (34), contre lequel un côté inférieur (36) de la composante pour ménisque (18) s'applique dans la position d'accouplement, dans lequel le dispositif d'endoprothèse d'articulation du genou (12) peut être transféré depuis la position de séparation par un mouvement relatif de la composante pour tibia (16) et de la composante pour ménisque (18) jusqu'à la position d'accouplement, **caractérisé en ce que** le dispositif d'endoprothèse d'articulation du genou (12) comprend un équipement d'accouplement (44) pour l'accouplement de la composante pour tibia (16) et de la composante pour ménisque (18) dans la position d'accouplement, dans lequel l'équipement d'accouplement (44) est conçu de sorte que, lors du transfert du dispositif d'endoprothèse d'articulation du genou (12) depuis la position de séparation jusqu'à la position d'accouplement, la composante pour tibia (16) et la composante pour ménisque (18) puissent être déplacées relativement l'une par rapport à l'autre depuis la position de séparation jusqu'à une position intermédiaire dans une direction (46) parallèle au plan d'application (34) , et en allant l'une vers l'autre depuis la position intermédiaire dans une direction (48) transversale, en particulier perpendiculaire, au plan d'application (34) jusqu'à la position d'accouplement.

2. Dispositif d'endoprothèse d'articulation du genou selon la revendication 1, **caractérisé en ce que** l'équipement d'accouplement (44) comprend au moins un premier élément d'accouplement (50) et au moins un deuxième élément d'accouplement (52), **en ce que** le au moins un premier élément d'accouplement (50) est agencé ou conçu contre la composante pour tibia (16) ou contre la composante pour ménisque (18), **en ce que** le au moins un deuxième élément d'accouplement (52) est agencé ou conçu contre la composante pour ménisque (18) ou contre la composante pour tibia (16), **en ce que** le au moins un premier élément d'accouplement (50) et le au moins un deuxième élément d'accouplement (52) ne sont pas en prise dans la position de séparation et **en ce que** le au moins un premier élément d'accouplement (50) et le au moins un deuxième élément d'accouplement (52) sont en prise dans la position d'accouplement.

3. Dispositif d'endoprothèse d'articulation du genou selon la revendication 2, **caractérisé en ce que** le au moins un premier élément d'accouplement (50) est conçu sous la forme d'une saillie d'accouplement (54), **en ce que** le au moins un deuxième élément d'accouplement (52) est conçu sous la forme d'un logement d'accouplement (56) avec un évidement d'accouplement (58), **en ce que** le logement d'accouplement (56) est conçu pour le logement de la saillie d'accouplement (54) dans la position intermédiaire et **en ce que** l'évidement d'accouplement (58) est conçu pour le logement d'au moins une partie de la saillie d'accouplement (54) dans la position d'accouplement,
dans lequel en particulier
a) l'évidement d'accouplement (58)
- est ouvert dans une direction perpendiculaire au plan d'application (34)
et/ou
- est ouvert exclusivement dans une direction perpendiculaire au plan d'application (34)
et/ou
b) la composante pour ménisque (18) comprend l'évidement d'accouplement (58) qui est ouvert pointant dans la direction vers la composante pour tibia (16) pour l'introduction de la au moins une partie de la saillie d'accouplement (54) à la suite d'un mouvement relatif depuis la position intermédiaire jusqu'à la position d'accouplement dans la direction transversale, en particulier perpendiculaire, au plan d'application (34).

4. Dispositif d'endoprothèse d'articulation du genou selon la revendication 3, **caractérisé en ce que** la composante pour ménisque (18) présente une ouverture d'introduction (60) pour l'introduction de la saillie d'accouplement (54) jusque dans le logement d'accouplement (56) de la composante pour ménisque (18),
dans lequel en particulier la saillie d'accouplement (54) présente un côté supérieur de saillie d'accouplement (62) pointant en s'éloignant du plan d'application (34), dans lequel l'ouverture d'introduction (60) définit une surface de délimitation supérieure (64) et une surface de délimitation inférieure (66) qui se déroulent parallèlement au côté inférieur (42) de la composante pour ménisque (18), dans lequel une distance (68) de la surface de délimitation inférieure (66) par rapport au côté inférieur (42) de la composante pour ménisque (18) est inférieure à une distance (70) de la surface de délimitation supérieure (64) par rapport au côté inférieur (42) de la composante pour ménisque (18) et dans lequel la distance (68) de la surface de délimitation inférieure (66) par rapport au côté inférieur (42) de la composante pour ménisque est inférieure à une distance (72) du côté supérieur de saillie d'accouplement (62) par rapport au plan d'application (34).

5. Dispositif d'endoprothèse d'articulation du genou selon la revendication 4, **caractérisé en ce que** l'ouverture d'introduction (60) définit une largeur d'introduction (74), **en ce que** la saillie d'accouplement (54) définit une largeur de saillie d'accouplement (76) et **en ce que** la largeur de saillie d'accouplement (76) correspond au moins à la largeur d'introduction (74),
dans lequel en particulier, la largeur de saillie d'accouplement (76) est supérieure à la largeur d'introduction (74).

6. Dispositif d'endoprothèse d'articulation du genou selon la revendication 5, **caractérisé en ce qu'**au moins des premiers et/ou deuxièmes éléments d'accouplement (104, 106) mobiles sont agencés ou conçus contre la saillie d'accouplement (54) et/ou contre l'ouverture d'introduction (60) et **en ce que** les éléments d'accouplement (104, 106) peuvent être déplacés lors du passage depuis la position de séparation à la position intermédiaire pour une réduction temporaire de la largeur de saillie d'accouplement (76) et/ou pour l'agrandissement de la largeur d'introduction (74),
dans lequel en particulier les au moins un premier et/ou deuxième éléments d'accouplement (104, 106) sont agencés ou conçus de façon à pouvoir être déplacés parallèlement au plan d'application (34).

7. Dispositif d'endoprothèse d'articulation du genou selon la revendication 6, **caractérisé en ce que** les au moins un premier et/ou deuxième éléments d'accouplement (104, 106) peuvent être déviés, à l'encontre de l'action d'un équipement de rappel (118), depuis une position de base d'élément d'accouplement, dans laquelle l'équipement de rappel (118) n'exerce aucune force de rappel sur les au moins un premier et/ou deuxième éléments d'accouplement (104, 106), jusqu'à une position de déviation d'élément d'accouplement.

8. Dispositif d'endoprothèse d'articulation du genou selon la revendication 7, **caractérisé en ce que** l'équipement de rappel (118) comprend au moins un élément de rappel (120, 122) et **en ce que** le au moins un élément de rappel (120, 122) est attribué à le au moins un premier et/ou deuxième élément d'accouplement (104, 106), dans lequel, en particulier
a) le au moins un premier et/ou deuxième élément d'accouplement (104, 106) comprend le au moins un élément de rappel (120, 122)
et/ou
b) un élément de rappel (102, 122) est respectivement attribué à chacun des au moins un premier et/ou deuxième éléments d'accouplement (104, 106)
et/ou
c) le au moins un élément de rappel (120, 122) est conçu sous la forme d'un élément de ressort (124, 126).

9. Dispositif d'endoprothèse d'articulation du genou selon l'une des revendications 3 à 8, **caractérisé en ce que** la saillie d'accouplement (54) comprend au moins un organe d'accouplement (98), **en ce que** le logement d'accouplement (56) comprend au moins un logement d'organe d'accouplement (160) et **en ce que** dans la position d'accouplement le au moins un organe d'accouplement (98) est en prise dans le au moins un logement d'organe d'accouplement,
dans lequel en particulier
a) le au moins un organe d'accouplement (98) est agencé ou conçu à distance par rapport à le au moins un élément d'accouplement (104, 106)
et/ou
b) le au moins un organe d'accouplement (98) est à distance dans une direction transversale, en particulier perpendiculaire, au plan d'application (34) en s'éloignant du au moins un élément d'accouplement (104, 106), en particulier en s'éloignant du plan d'application (34),
et/ou
c) le logement d'organe d'accouplement (160) est conçu sous la forme d'une rainure de logement d'organe d'accouplement (158) ouverte pointant dans la direction vers le plan d'application (34).

10. Dispositif d'endoprothèse d'articulation du genou selon l'une des revendications 3 à 9, **caractérisé en ce que**
a) la saillie d'accouplement (54) comprend au moins une première section (100), laquelle s'étend en s'éloignant directement à partir du côté supérieur (32) de la composante pour tibia (16), et une deuxième section (102) se raccordant à la première section (100), et **en ce qu'**une extension maximale de la première section (100) dans un plan parallèle au plan d'application (34) est inférieure à une extension maximale de la deuxième section (102) dans un plan parallèle au plan d'application (34),
dans lequel en particulier une contre-dépouille (132) ouverte parallèlement au plan d'application (34) est conçue entre le côté supérieur (32) de la composante pour tibia (16) et la deuxième section (102) dans la zone de la première section (100), en particulier sous la forme d'une rainure (90) périphérique au moins par sections, en particulier complètement, pointant en s'éloignant de l'axe de rotation (130)
et/ou
b) le logement d'accouplement (56) présente au moins une contre-dépouille (134) ouverte parallèlement au côté inférieur (42) de la composante pour ménisque (18) et pointant dans la direction vers l'axe de rotation (130), en particulier sous la forme d'une rainure (136) périphérique au moins par sections, en particulier complètement, pointant vers l'axe de rotation (130).

11. Dispositif d'endoprothèse d'articulation du genou selon l'une des revendications précédentes, **caractérisé en ce que** la composante pour ménisque (18) et la composante pour tibia (16) dans la position d'accouplement peuvent être déplacées, en particulier tournées et/ou coulissées, l'une par rapport à l'autre.

12. Dispositif d'endoprothèse d'articulation du genou selon l'une des revendications précédentes, **caractérisé en ce que** l'équipement d'accouplement (44) forme un équipement de palier rotatif (128) pour un montage en rotation de la composante pour ménisque (18) et de la composante pour tibia (16) l'une par rapport à l'autre dans la position d'accouplement,
dans lequel en particulier l'équipement de palier rotatif (128) comprend le au moins un premier élément d'accouplement (50) et le au moins un deuxième élément d'accouplement (52).

13. Dispositif d'endoprothèse d'articulation du genou selon l'une des revendications 2 à 12, **caractérisé en ce que** le au moins un premier élément d'accouplement (50) et le au moins un deuxième élément d'accouplement (52) sont conçus à symétrie de rotation ou essentiellement à symétrie de rotation et définissent un axe de rotation (130) par rapport auquel la composante pour ménisque (18) et la composante pour tibia (16) sont montés l'une par rapport à l'autre de façon à pouvoir tourner l'une par rapport à l'autre dans la position d'accouplement,
dans lequel en particulier
a) l'axe de rotation (130) se déroule transversalement, en particulier perpendiculairement, au plan d'application (34)
et/ou
b) les au moins un premier et/ou deuxième éléments d'accouplement (104, 106) sont agencés ou conçus de façon à pouvoir être déplacés et/ou pivotés en s'éloignant de l'axe de rotation (130) ou en allant vers l'axe de rotation (130)
et/ou
c) la rainure de logement d'organe d'accouplement (158) est agencée ou conçue de manière à entourer concentriquement l'axe de rotation (130).

14. Dispositif d'endoprothèse d'articulation du genou selon l'une des revendications précédentes, **caractérisé en ce que**
a) le dispositif d'endoprothèse d'articulation du genou (12) comprend un équipement de limitation de rotation (138) pour la limitation d'une rotation de la composante pour ménisque (18) et de la composante pour tibia (16) l'une par rapport à l'autre dans la position d'accouplement,
dans lequel en particulier l'équipement de limitation de rotation (138) comprend au moins une première surface de butée (142, 144) agencée ou conçue contre la composante pour tibia (16) et au moins une deuxième surface de butée (146, 148) coopérant avec celle-ci et agencée ou conçue contre la composante pour ménisque (18), et dans lequel la au moins une première surface de butée (142, 144) et la au moins une deuxième surface de butée (146, 148) s'appliquent l'une contre l'autre dans une position de rotation déviée depuis une position de base selon un angle de rotation maximal,
dans lequel en outre en particulier la au moins une première surface de butée (142, 144) et la au moins une deuxième surface de butée (146, 148) se déroulent transversalement, en particulier perpendiculairement, au plan d'application (34),
et/ou
b) la composante pour ménisque (18) présente un côté supérieur de composante pour ménisque (28), lequel présente des surfaces d'articulation du ménisque (24, 28) médiales et latérales.

15. Endoprothèse d'articulation du genou (10) comprenant un dispositif d'endoprothèse d'articulation du genou (12) et une composante pour fémur (14) coopérant avec celui-ci, **caractérisée en ce que** le dispositif d'endoprothèse d'articulation du genou (12) est conçu sous la forme d'un dispositif d'endoprothèse d'articulation du genou (12) selon l'une des revendications précédentes,
dans laquelle en particulier la composante pour fémur (14) comprend des surfaces d'articulation du fémur (20, 22) médiales et latérales pointant dans la direction vers la composante pour ménisque (18), lesquelles, dans une position d'implantation de l'endoprothèse d'articulation du genou (10), s'appliquent contre des surfaces d'articulation du ménisque (24, 26) médiales et latérales de la composante pour ménisque (18) et coopèrent avec celles-ci lors d'un mouvement relatif de la composante pour fémur (14) et de la composante pour ménisque (18).
